# EUROPÄISCHE PATENTSCHRIFT

(11) **EP 2 818 163 B1**
(45) Veröffentlichungstag und Bekanntmachung des Hinweises auf die Patenterteilung: **26.06.2024**
(21) Anmeldenummer: 14182601.6
(22) Anmeldetag: 25.09.2012
(51) Int. Cl.: A61K 31/164, A61K 31/525, A61K 31/4174, A61K 31/415, A61K 47/00, A61K 9/00, A61P 11/02

(54) **Zusammensetzung für die nasale Applikation mit verbesserter Stabilität**
Composition for nasal application with improved stability
Composition pour l'application nasale ayant une stabilité améliorée

(30) Priorität: 30.12.2011 DE 102011122588; 20.03.2012 DE 102012005452
(43) Veröffentlichungstag der Anmeldung: 31.12.2014
(62) Teilanmeldung aus: 12766592.5
(73) Patentinhaber: Maria Clementine Martin Klosterfrau Vertriebsgesellschaft mbH, 50670 Köln (DE)
(72) Erfinder: Greve, Harald, 40545 Düsseldorf (DE)
(74) Vertreter: Von Rohr Patentanwälte Partnerschaft mbB

(56) Entgegenhaltungen:
- EP-A2- 0 773 022
- EP-A2- 1 532 986
- WO-A1-2005/018601
- DE-A1- 10 000 612
- DE-U1-202008 012 494
- Cassella Med: "nasic für Kinder", , 1. Juni 2008 (2008-06-01), Seiten 1-2, XP055041369, Gefunden im Internet: URL:http://shop.schwan-apotheke.de/media/b eipackzettel/1356124.pdf [gefunden am 2012-10-17]
- Claudia Sikora: "Einfluss von Rezepturverbesserungen auf die Zytotoxizität von Nasalia", , 14. Juli 2006 (2006-07-14), XP055108889, Gefunden im Internet: URL:http://ub-ed.ub.uni-greifswald.de/opus /volltexte/2006/128/pdf/sikora_claudia.pdf [gefunden am 2014-03-19]

## Beschreibung

Die vorliegende Erfindung betrifft das Gebiet der Behandlung von Rhinitiden.

Insbesondere betrifft die vorliegende Erfindung eine pharmazeutische Zusammensetzung mit verbesserter Stabilität, welche sich für die topische intranasale Applikation für die Behandlung von Rhinitiden eignet, und die Verwendung dieser Zusammensetzung sowie eine diese Zusammensetzung enthaltende Applikationsvorrichtung.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen, allergischen oder pseudoallergischen Ursprungs sein kann. Am Häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen der Rhinitis, beispielsweise die *Rhinitis acuta, Rhinitis atrophicans, Rhinitis allergica, Rhinitis hypertrophica, Rhinitis medicamentosa, Rhinitis pseudomembranacea, Rhinitis sicca* und *Rhinitis vasomotorica.*

Bei der sogenannten akuten Rhinitis (*Rhinitis acuta*)*,* d. h. dem gewöhnlichen Schnupfen, handelt es sich in der Regel um einen im Allgemeinen harmlosen Infekt der Nasenschleimhäute und damit um eine infektiöse Rhinitis, welche durch eine Vielzahl von Viren (insbesondere Rhinoviren und/oder Adenoviren) ausgelöst werden kann; Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: *"Rhinitis", "Rhinitis allergica", "Rhinitis atrophicans", "Rhinitis hyperplastica", "Rhinitis pseudomembranacea", "Rhinitis sicca"* und *"Rhinitis vasomotorica".*

Da es also eine Vielzahl verschiedener Typen von Viren gibt, welche eine Rhinitis auslösen können, und da eine Vielzahl von Ursachen für das Auftreten einer Rhinitis existieren, können Rhinitiden, insbesondere akute Rhinitiden, im Allgemeinen nicht kausal, sondern nur symptomatisch, vorzugsweise topisch, behandelt werden, zumeist unter Anwendung von Sympathomimetika (synonym auch als sogenannte sogenannter "Abschweller" oder "Dekongestiva" bezeichnet), vorzugweise alpha-Sympathomimetika, wie Xylometazolin und Oxymetazolin oder deren physiologisch verträgliche Salze.

Diese Sympathomimetika führen aufgrund ihrer vasokonstriktorischen Eigenschaften nach lokaler bzw. topischer Anwendung in der Nase zwar zu einer Nasenschleimhautabschwellung, bewirken jedoch bei wiederholten Anwendung oftmals eine Austrocknung der Nasenschleimhäute, einhergehend mit entzündlichen Irritationen der Nasenschleimhäute - was nicht selten zu einer erhöhten Infektionsgefahr führt, da die Nasenschleimhäute im ausgetrockneten und entzündeten Zustand nicht mehr ihre Schutz- und Filterfunktionen im vollen Umfang aufrechterhalten können und folglich Krankheitserreger ungehindert in die Atemwege gelangen können.

Zudem sind diese Sympathomimetika, insbesondere Xylometazolin und Oxymetazolin sowie deren physiologisch verträglichen Salze, nicht unbegrenzt in wässriger Lösung lagerbar, so dass unerwünscht und unkontrolliert Abbauprodukte dieser Wirkstoffe entstehen können, welche dann einer Anwendung entsprechender wässriger Lösungen dieser Wirkstoffe entgegenstehen können.

So ist es beispielsweise für das alpha-Sympathomimetikum Xylometazolinhydrochlorid bekannt, dass es in wässriger Lösung hydrolytisch gespalten werden kann. Wie die nachfolgende Reaktionsgleichung zeigt, wird Xylometazolinhydrochlorid (1) in wässriger Lösung hydrolytisch unter Öffnung des Imidazolinrings gespalten, so dass als Abbauprodukt das Amid (2) gebildet wird, welches in der Monographie des Europäischen Arzneibuchs (Ph. Eur.) auch als sogenannte "Verunreinigung A" bezeichnet wird:

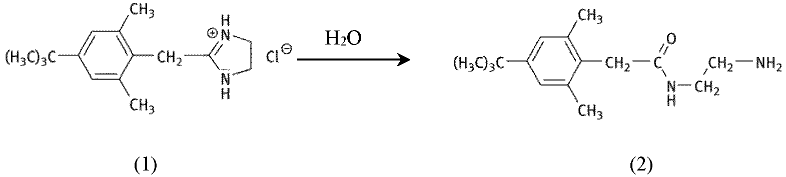

Für das Oxymetazolinhydrochlorid gilt ein analoger Mechanismus, welcher durch die folgende Reaktionsgleichung wiedergegeben werden kann, wobei das Oxymetazolinhydrochlorid (3) hydrolytisch zu dem Amid (4) als Abbauprodukt umgewandelt wird:

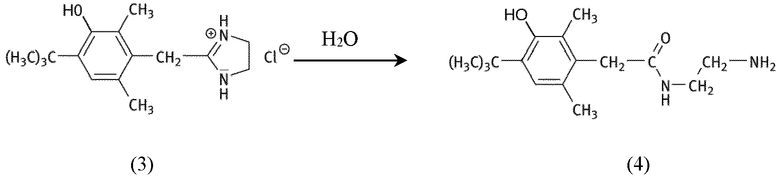

Um einem unerwünschten Abbau der Abschweller auf Sympathomimetikumbasis, insbesondere auf Basis von Xylometazolin und/oder Oxymetazolin sowie deren Salzen, entgegenzuwirken, wurden im Stand der Technik verschiedene Maßnahmen ergriffen, welche sich aber als unzulänglich erwiesen haben bzw. mit unerwünschten Nachteilen verbunden sind:
So führt die Zugabe externer Stabilisatoren oftmals zu einer nur unzureichenden Stabilisierung dieser Wirkstoffe. Eine Zugabe komplexierender Salze, insbesondere von Zinksalzen, führt zwar zu einer gewissen Stabilitätsverbesserung (vgl. DE 103 37 186 A1 und WO 2005/018601 A1), führt jedoch bei nasaler Applikation zu einer unerwünschten Reizung der Nasenschleimhäute infolge der Zinksalze, einhergehend mit entzündlichen Irritationen.

Daher können die im Stand der Technik aufgezeigten Versuche bzw. Lösungsansätze zur Stabilisierung der vorgenannten sympathomimetikabasierten Wirkstoffe in wässrigen Systemen nur als praktizierter Kompromiss in Ermangelung einer besseren Alternative angesehen werden. Zudem berücksichtigen zu vorgenannten Lösungsansätze nicht den austrocknenden Effekt dieser Wirkstoffe in Bezug auf die Nasenschleimhäute.

Um andererseits einer Austrocknung der Nasenschleimhäute durch die Anwendung von alpha-Sympathomimetika entgegenzuwirken, wurde im Stand der Technik die kombinierte Verabreichung von Pantothenol und/oder Pantothensäure (vgl. die zu derselben Patentfamilie gehörenden Druckschriften DE 195 41 919 A1 bzw. EP 0 773 022 A2, DE 195 49 421 A1 und EP 1 663 141 B1) bzw. Hyaluronsäure (vgl. DE 103 56 248 A1 bzw. EP 1 532 986 A2) vorgeschlagen, da diese Wirkstoffe (d. h. Pantothenol bzw. Pantothensäure bzw. Hyaluronsäure und deren jeweils physiologisch verträgliche Salze) einer Austrocknung der Nasenschleimhäute durch die Anwendung der alpha-Sympathomimetika in effizienter Weise entgegenwirken. In diesem Zusammenhang hat sich insbesondere die Anwendung von Pantothenol bzw. Pantothensäure bewährt.

Das Dokument gemäß Cassella Med: "nasic® für Kinder", 1. Juni 2008, Seiten 1 bis 2, betrifft die zugrundeliegende Fachinformation zu dem Marktprodukt "nasic^{®} für Kinder".

Die DE 20 2008 012 494 U1 betrifft eine pharmazeutische Zubereitung, insbesondere zur prophylaktischen bzw. kurativen Behandlung der trockenen Nasenschleimhaut bzw. von Rhinitiden, wobei die Zusammensetzung als Wirkstoffkomponente Ectoin in Kombination mit Pantothenol enthalten soll.

Die DE 100 00 612 A1 betrifft Flüssigpräparate für Augen- und Nasentropfen bzw. ein Nasenspray ohne Konservierungsmittel, wobei das Präparat gemäß D6 zwingend Kochsalz bzw. NaCl aufweisen soll, wobei die Zusammensetzung gleichermaßen einen Vasokonstriktor in Form von Oxymetazolinhydrochlorid bzw. Xylometazolinhydrochlorid aufweisen kann.

Das Dokument gemäß der Dissertationsschrift "Einfluss von Rezepturverbesserung auf die Zytoxizität von Nasalia, Claudia Sikora" betrifft allgemeine Ausführungen bzw. Untersuchungen zu einer Vielzahl von nasal anwendbaren Arzneimitteln und Medizinprodukten bzw. von Nasalia, wobei in diesem Zusammenhang maßgeblich auf den Einfluss von Konservierungsmitteln insbesondere im Hinblick auf deren Verträglichkeit bei der Anwendung der untersuchten Zusammensetzungen abgestellt wird.

Insbesondere der Wirkstoff Pantothenol (synonym auch als "Dexpanthenol" bezeichnet) kann jedoch in wässriger Lösung hydrolytisch gemäß der nachfolgenden Reaktionsgleichung gespalten werden, wobei sich aus dem Wirkstoff Panthenol (3) in saurer Lösung - neben dem Ammoniumsalz des 3-Aminopropanols (7) - das Lacton D-Pantolacton (6) als Abbauprodukt bildet, während in alkalischer Lösung - neben 3-Aminopropanol (5) - das Salz der Pantosäure (4) gebildet wird:

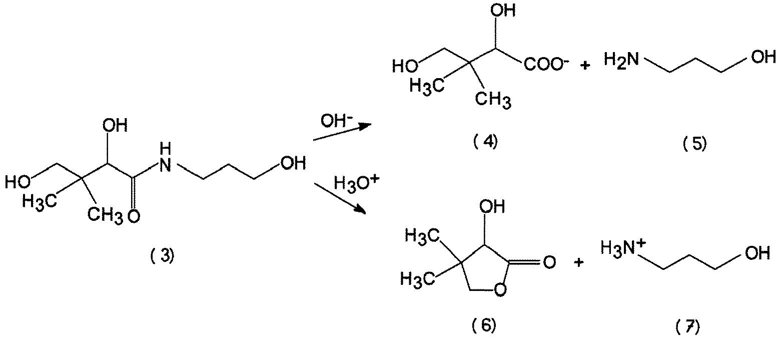

In gemeinsamer wässriger Lösung von alpha-Sympathomimetika, insbesondere auf Basis von Xylometazolin und/oder Oxymetazolin, einerseits und Panthenol bzw. Pantothensäure andererseits ist eine Stabilisierung der einzelnen Inhaltsstoffe besonders diffizil, da die einzelnen Wirkstoffe (d. h. Xylometazolin bzw. Oxymetazolin einerseits und Panthenol bzw. Dexpanthenol andererseits) in unterschiedlichen pH-Bereichen ihr Stabilitätsoptimum aufweisen.

Das der vorliegenden Erfindung zugrundeliegende Problem besteht daher in der Bereitstellung einer für die topische, insbesondere nasale, bevorzugt intranasale Applikation, insbesondere für die Behandlung von Rhinitiden, geeigneten Zusammensetzung, insbesondere pharmazeutischen Zusammensetzung, welche die zuvor geschilderten Nachteile des Standes der Technik zumindest weitgehend vermeidet oder aber wenigstens abschwächt, insbesondere eine verbesserte Stabilität (z. B. Lagerstabilität) aufweist. Insbesondere soll eine solche Zusammensetzung auch nach längerer Lagerung keine bzw. keine signifikanten Mengen an Abbauprodukten der Inhaltsstoffe, insbesondere keine signifikanten Mengen an Abbauprodukten des Xylometazolins und/oder Oxymetazolins und des Panthenols bzw. der Pantothensäure, aufweisen.

Zur Lösung des zuvor geschilderten Problems schlägt die vorliegende Erfindung - gemäß einem ersten Aspekt der vorliegenden Erfindung - eine pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem zweiten Aspekt der vorliegenden Erfindung - eine die erfindungsgemäße Zusammensetzung enthaltende Applikationsvorrichtung gemäß dem betreffenden Vorrichtungsanspruch; weitere, insbesondere vorteilhafte Ausgestaltungen der erfindungsgemäßen Applikationsvorrichtung sind Gegenstand des diesbezüglichen Unteranspruchs.

Weiterhin betrifft die vorliegende Erfindung - gemäß einem weiteren und dritten Aspekt der vorliegenden Erfindung - die erfindungsgemäße Zusammensetzung zur Verwendung nach der vorliegenden Erfindung, wie sie in den entsprechenden die Zusammensetzung zur Verwendung betreffenden Ansprüchen definiert ist.

Schließlich betrifft die vorliegende Erfindung - gemäß einem weiteren und vierten Aspekt der vorliegenden Erfindung - das erfindungsgemäße Verfahren zur Stabilisierung eines Wirkstoffes a) und eines Wirkstoffes b) in gemeinsamer wässriger Lösung einer pharmazeutischen Zusammensetzung, wie es in dem entsprechenden Verfahrensanspruch definiert ist.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine pharmazeutische Zusammensetzung mit verbesserter Stabilität, welche für die topische intranasale Applikation für die Behandlung von Rhinitiden geeignet ist,
wobei die Zusammensetzung als wässrige Zusammensetzung vorliegt und
wobei die Zusammensetzung enthält:
   a) mindestens ein imidazolinbasiertes alpha-Sympathomimetikum oder dessen physiologisch unbedenkliches Salz, wobei das imidazolinbasierte alpha-Sympathomimentikum ausgewählt ist aus Xylometazolin;
      und
   b) Pantothenol (Dexpanthenol);
wobei der pH-Wert der Zusammensetzung im Bereich von 5,2 bis 5,9 eingestellt und/oder konstantgehalten ist, wobei die Einstellung und/oder Konstanthaltung des pH-Werts der Zusammensetzung mittels mindestens eines chemischen Puffersystems erfolgt, wobei als chemisches Puffersystem ein Dihydrogenphosphat/Monohydrogenposphat-Puffersystem ("H₂PO₄⁻/HPO₄²⁻-Puffer(system)" bzw. "Phosphatpuffer(system)") eingesetzt ist, wobei das Dihydrogenphosphat/Monohydrogenposphat-Puffersystem mit einem Dihydrogenphosphat/Monohydrogenposphat-Molverhältnis im Bereich von 6 : 1 bis 110 : 1 eingesetzt ist;
wobei die Zusammensetzung einen Gehalt an Abbauprodukt(en) des Wirkstoffs a), insbesondere Verunreinigung A, von höchstens 5 Gew.-%, bezogen auf den Wirkstoff a), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten; und
wobei die Zusammensetzung einen Gehalt an Abbauprodukt(en) des Wirkstoffs b), insbesondere Aminopropanol und/oder D-Pantolacton, von jeweils höchstens 5 Gew.-%, bezogen auf den Wirkstoff b), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten.

Denn, wie die Anmelderin vollkommen überraschend herausgefunden hat, gelingt es bei Einstellung bzw. Konstanthaltung dieses sehr engen pH-Bereichs von 5,2 bis 5,9, einerseits das imidazolinbasierte alpha-Sympathomimetikum und andererseits auch das Pantothenol (Dexpanthenol) bzw. die Pantothensäure in effizienter Weise zu stabilisieren, ohne dass eine nennenswerte Hydrolyse oder ein anderweitiger Abbau dieser beiden Wirkstoffe bzw. Wirkstoffkomponenten eintritt.

Mit anderen Worten lässt sich unter Einhaltung des vorgenannten, sehr engen pH-Regimes von 5,2 bis 5,9 eine Stabilisierung beider Wirkstoffe a) und b) auch in gemeinsamer wässriger Lösung erreichen, insbesondere ohne den zusätzlichen Einsatz von Stabilisatoren oder Konservierungsmittel.

Dies ist umso überraschender, als in diesem leicht sauren pH-Bereich von 5,2 bis 5,9 sowohl ein hydrolytischer Abbau des alpha-Sympathomimetikums als auch des Pantothenols bzw. der Pantothensäure zu erwarten gewesen wäre, da dieses Phänomen in Lösungen der Einzelwirkstoffe beobachtet wird. Überraschenderweise tritt ein hydrolytischer Abbau der beiden Wirkstoffkomponenten a) und b) jedoch nicht ein, sofern einerseits die beiden vorgenannten Wirkstoffkomponenten a) und b) in gemeinsamer Lösung vorliegen und andererseits dieser gezielt und eng ausgewählte pH-Wertbereich von 5,2 bis 5,9 konstantgehalten bzw. eingestellt wird. Das Auffinden dieses Effekts, insbesondere im Hinblick auf die Stabilisierung der Zusammensetzung, ist vollkommen überraschend und war angesichts des zuvor geschilderten Standes der Technik in dieser Weise nicht zu erwarten. Hierauf wird im Nachfolgenden noch im Detail eingegangen werden.

Erfindungsgemäß erfolgt im Rahmen der vorliegenden Erfindung die Bestimmung bzw. Messung des pH-Wertes mit dem Fachmann an sich bekannten bzw. geläufigen oder aber unter standardisierten Methoden, insbesondere bei 20 °C und Atmosphärendruck (1.013,25 mbar). Bekanntermaßen bezeichnet der sogenannte pH-Wert eine dimensionslose Maßzahl, welche nach DIN 19260: 1971-03 als der negative dekadische Logarithmus der Wasserstoffionen-Aktivität definiert wird. Im Rahmen der vorliegenden Erfindung bezieht sich die Angabe des pH-Werts insbesondere auf die Bestimmungsmethode gemäß DIN 19266: 2000-01, welche die ältere DIN 19266: 1979-08 ersetzt. Für weitergehende Einzelheiten zum Begriff des pH-Werts und seiner Messung und Bestimmung kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "*pH*"*,* sowie auf die dort referierte Literatur, wobei die gesamte vorgenannte Literaturstelle mit der dort referierten Literatur hiermit durch Bezugnahme eingeschlossen sind.

Gemäß einer bevorzugten Ausführungsform der vorliegenden Erfindung ist die Komponente a) bzw. das imidazolinbasierte alpha-Sympathomimetikum ausgewählt aus Xylometazolin in Form von dessen physiologisch unbedenklichen Salzen, besonders bevorzugt in Form von dessen Hydrochloridsalz (Xylometazolinhydrochlorid).

Die Komponente b) der erfindungsgemäßen Zusammensetzung ist Pantothenol (Dexpanthenol).

Gemäß einer erfindungsgemäß bevorzugten Ausführungsform enthält die erfindungsgemäße Zusammensetzung
a) Xylometazolin oder deren physiologisch unbedenkliche Salze, besonders bevorzugt in Form von dessen Hydrochloridsalz (Xylometazolinhydrochlorid);
   und
b) Pantothenol (Dexpanthenol).

Wie nachfolgend noch geschildert, bewirkt bei topischer, insbesondere nasaler, bevorzugt intranasaler Applikation der erfindungsgemäßen Zusammensetzung, insbesondere bei der Behandlung von Rhinitiden, die Wirkkomponente a) aufgrund ihrer vasokonstriktorischen Eigenschaften eine Abschwellung der Nasenschleimhäute, während die Wirkkomponente b) eine Austrocknung und entzündlichen Irritation der Schleimhäute, insbesondere infolge der Anwendung des alpha-Sympathomimetikums, entgegenwirkt.

Die Menge an Komponente a) in der erfindungsgemäßen Zusammensetzung kann in weiten Bereichen variieren. Um eine ausreichende Wirkung der Komponente a) in der erfindungsgemäßen Zusammensetzung zu gewährleisten, ist es vorteilhaft, wenn die erfindungsgemäße Zusammensetzung die Komponente a), bezogen auf die erfindungsgemäße Zusammensetzung, in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält. Dennoch kann der Fachmann anwendungsbezogen oder einzelfallbedingt von den vorgenannten Mengenangaben abweichen, ohne dass er den Rahmen der vorliegenden Erfindung verlässt.

Gleichermaßen kann auch die Menge an der Komponente b) in der erfindungsgemäßen Zusammensetzung in weiten Bereichen variieren. Um eine ausreichende Wirkung durch die Komponente b) zu gewährleisten, ist es jedoch vorteilhaft, wenn die erfindungsgemäße Zusammensetzung die Komponente b), bezogen auf die erfindungsgemäße Zusammensetzung, in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält. Dennoch kann der Fachmann anwendungsbezogen oder einzelfallbedingt von den vorgenannten Mengenangaben abweichen, ohne dass er den Rahmen der vorliegenden Erfindung verlässt.

Für eine ausreichende Wirkung einerseits und eine zuverlässige Stabilisierung der beiden Komponenten a) und b) andererseits ist auch das Mengenverhältnis der Komponente a) zu der Komponente b) von Bedeutung. Insbesondere ist es in diesem Zusammenhang von Vorteil, wenn die erfindungsgemäße Zusammensetzung die Komponenten a) und b) in einem Mengenverhältnis von Komponente a) zu Komponente b) im Bereich von 1 : 10 bis 1 : 1.000, insbesondere 1 : 15 bis 1 : 500, vorzugsweise 1 : 20 bis 1 : 250, bevorzugt 1 : 25 bis 1 : 200, besonders bevorzugt 1 :30 bis 1 : 175, ganz besonders bevorzugt 1 : 40 bis 1 : 150, noch mehr bevorzugt 1 : 45 bis 1 : 125, enthält. Auf diese Weise werden Wirkeffizienz und Stabilität, insbesondere Lagerstabilität, in gleicher Weise gewährleistet. Dennoch ist es nicht ausgeschlossen, dass der Fachmann einzelfallbedingt oder anwendungsbezogen von den vorgenannten Wertebereichen abweichen kann, ohne dass er den Rahmen der vorliegenden Erfindung verlässt.

Erfindungsgemäß liegt die erfindungsgemäße Zusammensetzung als wässrige Zusammensetzung vor. Insbesondere ist also die erfindungsgemäße Zusammensetzung wässrig basiert bzw. liegt die erfindungsgemäße Zusammensetzung wässrig formuliert vor, insbesondere in Form einer wässrigen Lösung oder wässrigen Solubilisierung.

Bevorzugterweise liegt somit die erfindungsgemäße Zusammensetzung als wässriges System, insbesondere als wässriges Einphasensystem, vorzugsweise als wässrige Lösung oder wässrige Solubilisierung, vor. Dabei weist die erfindungsgemäße Zusammensetzung somit einen Exzipienten oder Träger auf Wasserbasis auf.

Im Allgemeinen liegt die erfindungsgemäße Zusammensetzung als klare, farblose wässrige Lösung vor. Dies hat zum einen den Vorteil einer vereinfachten Applikation. Zum anderen lassen sich an einer klaren, farblosen wässrigen Lösung Veränderungen ohne Weiteres wahrnehmen.

Wie zuvor dargelegt, gelingt die Stabilisierung der erfindungsgemäßen Zusammensetzung, insbesondere der Wirkstoffe a) und b) der erfindungsgemäßen Zusammensetzung, durch Einstellung und/oder Konstanthaltung des pH-Werts der erfindungsgemäßen Zusammensetzung im Bereich von 5,2 bis 5,9. Besonders gute Ergebnisse, insbesondere im Hinblick auf die Stabilität, werden erhalten, wenn der pH-Wert der erfindungsgemäßen Zusammensetzung im Bereich von 5,2 bis 5,9 eingestellt und/oder konstantgehalten wird.

Erfindungsgemäß erfolgt die Einstellung und/oder Konstanthaltung des pH-Werts der erfindungsgemäßen Zusammensetzung mittels mindestens eines chemischen Puffersystems, insbesondere mit Hilfe von Puffersalz(en).

Zum Begriff des chemischen Puffers der Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "*Puffer",* sowie auf die dort referierte Literatur.

Mit anderen Worten enthält die erfindungsgemäße Zusammensetzung also mindestens ein chemisches Puffersystem, insbesondere Puffersalz(e).

In diesem Zusammenhang hat es sich insbesondere bewährt, wenn zur Einstellung und/oder Konstanthaltung des pH-Werts der erfindungsgemäßen Zusammensetzung ein Dihydrogenphosphat/Monohydrogenposphat-Puffersystem verwendet wird, welches auch als "H₂PO₄⁻/HPO₄²⁻-Puffer(system)" bzw. "Phosphatpuffer(system)" bezeichnet wird; erfindungsgemäß besonders bevorzugt ist ein Alkalidihydrogenphosphat/Alkalimonohydrogenposphat-Puffersystem.

Denn, wie die Stabilitätsuntersuchungen der Anmelderin in überraschender Weise gezeigt haben, bewirkt nur ein solches Phosphatpuffersystem - gegenüber anderen möglichen einsetzbaren Puffersystemen - die angestrebte und zuverlässige Langzeitstabilisierung der erfindungsgemäßen Zusammensetzung auch über mehr als zwei Jahre. Mit anderen Puffersystemen dagegen, welche sich im vergleichbaren pH-Wertbereich einsetzen lassen, wie z. B. einem Kohlensäure/Bicarbonat-Puffersystem, einem Kohlensäure/Silikat-Puffersystem, einem Essigsäure/Acetat-Puffersystem, einem Citronensäure/Citrat-Puffersystem oder dergleichen, lassen sich derart gute Stabilitätsergebnisse nicht bzw. nicht immer zuverlässig erhalten. Ohne sich auf eine bestimmte Theorie festlegen zu wollen, lässt sich dieser Effekt des erfindungsgemäß eingesetzten Phosphatpuffersystems möglicherweise auf sekundäre Effekte, wie Komplexierungsreaktionen oder dergleichen, zurückführen.

Im Hinblick auf die angestrebte Langzeitstabilität bzw. Langzeitstabilisierung der erfindungsgemäßen Zusammensetzung hat es sich besonders bewährt, wenn das erfindungsgemäß zur Einstellung bzw. Konstanthaltung des pH-Werts verwendete Dihydrogenphosphat/Monohydrogenposphat-Puffersystem mit einem Dihydrogenphosphat/Monohydrogenposphat-Molverhältnis im Bereich von 6 : 1 bis 110 : 1, besonders bevorzugt im Bereich von 6 : 1 bis 105 : 1, ganz besonders bevorzugt im Bereich von 7 : 1 bis 100 : 1, eingesetzt wird. Mit anderen Worten enthält die erfindungsgemäße Zusammensetzung aus den vorgenannten Gründen das Dihydrogenphosphat/Monohydrogenposphat-Puffersystem bevorzugterweise mit einem Dihydrogenphosphat/Monohydrogenposphat-Molverhältnis im Bereich von 6 : 1 bis 110 : 1, besonders bevorzugt im Bereich von 6 : 1 bis 105 : 1, ganz besonders bevorzugt im Bereich von 7 : 1 bis 100 : 1.

Weiterhin kann es erfindungsgemäß vorgesehen sein, dass die erfindungsgemäße Zusammensetzung als weiteren Wirk- bzw. Inhaltsstoff c) mindestens ein Konservierungsmittel und/oder Desinfektionsmittel enthält. Das optional vorhandene Konservierungsmittel und/oder Desinfektionsmittel kann dabei insbesondere ausgewählt sein aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen. Besonders bevorzugt wird als Konservierungsmittel und/oder Desinfektionsmittel, sofern vorhanden, Benzalkoniumchlorid eingesetzt; bei Benzalkoniumchlorid handelt es sich um ein Gemisch aus Alkylbenzyldimethylammoniumchloriden, deren Alkylteil aus Cs-Cis-Ketten besteht, wobei Benzalkoniumchlorid insbesondere für seine desinfizierende und konservierende Wirkung bekannt ist. Sofern die Zusammensetzung ein Konservierungsmittel und/oder Desinfektionsmittel enthält, sollte die Menge an Konservierungsmittel und/oder Desinfektionsmittel, bezogen auf die erfindungsgemäße Zusammensetzung, 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, betragen.

Im Rahmen der vorliegenden Erfindung konnte die Anmelderin vollkommen unerwartet herausfinden, dass die Verwendung eines Konservierungsmittels und/oder Desinfektionsmittels, insbesondere Benzalkoniumchlorid, zu einer zusätzlichen Stabilisierung der Wirkstoffkomponenten a) und b) führt. Darüber hinaus hat die Verwendung eines Konservierungsmittels bzw. Desinfektionsmittels auch anwendungsbezogene Vorteile, da der desinfizierende Effekt des Konservierungs- bzw. Desinfektionsmittels die Wirkung der Wirkstoffkomponenten a) und b) unterstützt und darüber hinaus einer Keimbildung in der erfindungsgemäßen Zusammensetzung in effizienter Weise entgegengewirkt wird.

Weiterhin kann es vorgesehen sein, dass die erfindungsgemäße Zusammensetzung als weiteren Wirk- bzw. Inhaltsstoff d) mindestens ein vorzugsweise saures Glykosaminoglykan oder dessen physiologisch unbedenkliche Salze oder Derivate, insbesondere Hyaluronsäure oder deren physiologisch unbedenkliche Salze, enthält, insbesondere in einer Menge von 0,0001 bis 10 Gew.-%, insbesondere 0,001 bis 5 Gew.-%, vorzugsweise 0,01 bis 2 Gew.-%, bezogen auf die erfindungsgemäße Zusammensetzung. Wie die Anmelderin überraschenderweise herausgefunden hat, können Glykosaminoglykane, insbesondere Hyaluronsäure bzw. deren Salze, die Wirkung der übrigen Wirk- bzw. Inhaltsstoffe, insbesondere des Panthenols bzw. der Pantothensäure, unterstützen. Insbesondere können die Glykosaminoglykane, vorzugsweise die Hyaluronsäure, förderlich in Bezug auf die Linderung von Infektionen und Entzündungen der Nasenschleimhaut wirken und darüber hinaus eine Beschleunigung der Regeneration von entzündetem Gewebe bewirken.

Weiterhin kann es erfindungsgemäß vorgesehen sein, dass die erfindungsgemäße Zusammensetzung als weiteren Wirk- bzw. Inhaltsstoff e) Ectoin oder mindestens ein Ectoinderivat, insbesondere ein Hydroxyectoin, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung. Wie die Anmelderin überraschend herausgefunden hat, ist die Verwendung von Ectoin bzw. von Ectoinderivaten im Hinblick auf eine Beschleunigung der Regeneration entzündeter Nasenschleimhaut von Vorteil.

Des Weiteren kann es erfindungsgemäße vorgesehen sein, dass die erfindungsgemäße Zusammensetzung als weiteren Wirk- bzw. Inhaltsstoff f) Natriumchlorid enthält, insbesondere in einer Menge von 0,001 bis 5 Gew.-%, insbesondere 0,01 bis 2 Gew.-%, vorzugsweise 0,1 bis 1 Gew.-%, bezogen auf die Zusammensetzung. Natriumchlorid bewirkt insbesondere eine Befeuchtung der Nasenschleimhäute und somit eine Beschleunigung der Regeneration entzündeter Nasenschleimhäute.

Gleichermaßen kann es vorgesehen sein, dass die erfindungsgemäße Zusammensetzung mindestens einen weiteren Inhaltsstoff aufweist. Dieser weitere Inhaltsstoff kann insbesondere ausgewählt sein aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Des Weiteren ist die Osmolalität der erfindungsgemäßen Zusammensetzung von Bedeutung. Die Osmolalität der erfindungsgemäßen Zusammensetzung kann in weiten Bereichen variieren. Um eine gute Verträglichkeit der erfindungsgemäßen Zusammensetzung in Bezug auf die Nasenschleimhaut zu gewährleisten, ist es von Vorteil, wenn die Zusammensetzung nach der vorliegenden Erfindung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist. Auf diese Weise ist bei topischer Anwendung der erfindungsgemäßen Zusammensetzung auf die Nasenschleimhäute eine gute Verträglichkeit gewährleistet.

Für eine gute Handhabbarkeit der erfindungsgemäßen Zusammensetzung ist es weiterhin von Vorteil, wenn die erfindungsgemäße Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist.

Wie zuvor dargelegt, besitzt aufgrund der erfindungsgemäßen Maßnahmen, wie sie zuvor geschildert worden sind, die erfindungsgemäße Zusammensetzung eine ausgezeichnete Stabilität bzw. Langzeitstabilität, insbesondere Lagerstabilität. Insbesondere ist die erfindungsgemäße Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil.

Die Stabilität der erfindungsgemäßen Zusammensetzung lässt sich durch das Vorhandensein bzw. Gehalt der Abbauprodukte der Wirkstoffe a) und b) bestimmen und charakterisieren. Wie eingangs geschildert, ist das charakteristische Abbauprodukt von Xylometazolin bzw. Xylometazolinhydrochlorid die sogenannte Verunreinigung A. Wie eingangs gleichermaßen geschildert, sind die typischen Abbauprodukte des Wirkstoffs b), insbesondere von Pantothenol, das Aminopropanol bzw. dessen Ammoniumsalz und D-Pantolacton.

In diesem Zusammenhang enthält die erfindungsgemäße Zusammensetzung einen Gehalt an Abbauprodukt(en) des Wirkstoffs a), insbesondere Verunreinigung A, von höchstens 5 Gew.-%, insbesondere von höchstens 3 Gew.-%, vorzugsweise von höchstens 2 Gew.-%, besonders bevorzugt von höchstens 1 Gew.-%, bezogen auf den Wirkstoff a); dieser allenfalls äußerst geringe Gehalt an Abbauprodukt(en) des Wirkstoffs a) wird auch nach Lagerung der erfindungsgemäßen Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, noch eingehalten.

Weiterhin weist in diesem Zusammenhang die erfindungsgemäße Zusammensetzung einen Gehalt an Abbauprodukt(en) des Wirkstoffs b), insbesondere Aminopropanol und/oder D-Pantolacton, von jeweils höchstens 5 Gew.-%, insbesondere von jeweils höchstens 3 Gew.-%, vorzugsweise von jeweils höchstens 2 Gew.-%, besonders bevorzugt von jeweils höchstens 1 Gew.-%, bezogen auf den Wirkstoff b), auf; dieser allenfalls äußerst geringe Gehalt an Abbauprodukt(en) des Wirkstoffs b) wird auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, noch eingehalten.

Des Weiteren betrifft die vorliegende Erfindung - gemäß einem **zweiten** Aspekt der vorliegenden Erfindung - eine Applikationsvorrichtung, welche eine wie zuvor beschriebene Zusammensetzung nach der vorliegenden Erfindung enthält, wobei die erfindungsgemäße Applikationsvorrichtung insbesondere für die topische, insbesondere nasale, vorzugsweise intranasale Applikation geeignet ist und bevorzugt in Form eines Behältnisses mit Tropf- oder Sprüheinrichtung vorliegt.

Erfindungsgemäß bevorzugt ist es, wenn die erfindungsgemäße Applikationsvorrichtung eine Sprüheinrichtung zur vorzugsweise gleichförmigen Ausbringung der Zusammensetzung nach der vorliegenden Erfindung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Dabei ist es insbesondere vorgesehen, dass die erfindungsgemäße Applikationsvorrichtung ein Behältnis bzw. einen Vorratsbehälter mit einem Volumen im Bereich von 5 ml bis 100 ml, insbesondere 10 ml bis 50 ml, aufweist. Dieses Behältnis bzw. dieser Vorratsbehälter dient dann zur Aufnahme der erfindungsgemäßen Zusammensetzung.

Für weitergehende Einzelheiten zu der erfindungsgemäßen Applikationsvorrichtung kann auf die vorangehenden Ausführungen zu der erfindungsgemäßen Zusammensetzung verwiesen werden, welche im Hinblick auf die erfindungsgemäße Applikationsvorrichtung entsprechend gelten.

Weiterer Gegenstand der vorliegenden Erfindung - gemäß einem **dritten** Aspekt der vorliegenden Erfindung - ist die zuvor beschriebene Zusammensetzung nach der vorliegenden Erfindung zur Verwendung bei der prophylaktischen und/oder kurativen topischen Behandlung von Rhinitiden aller Art, insbesondere *Rhinitis acuta,* bzw. die Verwendung einer wie zuvor beschriebenen Zusammensetzung nach der vorliegenden Erfindung zur Herstellung eines Arzneimittels zur prophylaktischen und/oder kurativen topischen Behandlung von Rhinitiden aller Art, insbesondere *Rhinitis acuta.*

Insbesondere kann die erfindungsgemäße Zusammensetzung zur Verwendung bei der Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta,* eingesetzt werden.

Wie zuvor beschrieben, kann im Rahmen der erfindungsgemäßen Zusammensetzung zur Verwendung die erfindungsgemäße Zusammensetzung, wie zuvor beschrieben, intranasal appliziert werden. Dabei kann insbesondere die erfindungsgemäße Applikationseinrichtung, wie sie zuvor beschrieben worden ist, zur Anwendung kommen.

Für weitergehende Einzelheiten zu der erfindungsgemäßen Zusammensetzung zur Verwendung kann auf die vorangehenden Ausführungen zu den übrigen Erfindungsaspekten verwiesen werden, welche in Bezug auf die erfindungsgemäße Zusammensetzung zur Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele:

**1. Herstellungsbeispiele:**
Allgemeine Herstellvorschrift
Zur Herstellung von jeweils 1.000 g einer klaren wässrigen Lösung der erfindungsgemäßen Zusammensetzung wird in dem Fachmann an sich bekannter Weise vorgegangen: Zunächst wird bei Raumtemperatur und Umgebungsdruck in einem entsprechenden Glasgefäß mit Rühreinrichtung eine definierte Menge an gereinigtem Wasser (z. B. 300 ml bis 600 ml) vorgelegt und nachfolgend mit einer Lösung des gewählten Puffersystems auf einen vorgegeben pH-Wert im Bereich zwischen 5,2 und 5,9 (z. B. pH-Wert von ca. 5,5) unter anschließender Kontrolle des erreichten pH-Werts eingestellt. Anschließend wird hierzu die nachfolgend in den Rezepturbeispielen spezifizierte Dexpanthenolmenge hinzugegeben, und das Ganze wird dann durch gründliches Rühren klar gelöst. Der Lösung wird dann gegebenenfalls ein Konservierungs- bzw. Desinfektionsmittel, insbesondere Benzalkoniumchlorid, bevorzugt in Form einer wässrigen Lösung, zugesetzt und gleichfalls unter Rühren gelöst. Nachfolgend wird die gewünschte Xylometazolin- bzw. Oxymetazolinhydrochloridmenge zugesetzt, mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen entsprechenden Spezifikationen der Lösung werden auf die eingestellten bzw. vorgewählten Wertebereiche hin überprüft (z. B. Osmolalität: 400 bis 490 mosm/kg, relative Dichte bei 20 °C: 1,01 bis 1,05; mikrobiologische Reinheit und Sterilität; Ausschluss von Verunreinigungen, insbesondere Abbauprodukten der Inhalts- und Wirkstoffe). Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; 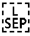 zur bestimmungsgemäßen Anwendung können mehrmals täglich ein bis drei Tropfen bzw. ein bis zwei Sprühstöße in jedes Nasenloch gegeben und aufgezogen werden. Ein anderer Teil der erhaltenen Lösung wird für die nachfolgend beschriebenen Stabilitätsuntersuchungen verwendet.
Nach dieser allgemeinen Herstellvorschrift werden die nachfolgend spezifizierten Rezepturen hergestellt.
Rezeptur 1 (Angabe pro 10 g Zusammensetzung, zur Anwendung bei Erwachsenen):

| **Inhaltsstoff** | **Menge/mg** | **Qualität** |
|---|---|---|
| Xylometazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.402,00 | Ph. Eur. |

Rezeptur 2 (Angabe pro 10 g Zusammensetzung, zur Anwendung bei Erwachsenen) (nicht erfindungsgemäß):

| **Inhaltsstoff** | **Menge/mg** | **Qualität** |
|---|---|---|
| Oxymetazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.402,00 | Ph. Eur. |

Rezeptur 3 (Angabe pro 10 g Zusammensetzung, zur Anwendung bei Kindern):

| **Inhaltsstoff** | **Menge/mg** | **Qualität** |
|---|---|---|
| Xylometazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.407,00 | Ph. Eur. |

Rezeptur 4 (Angabe pro 10 g Zusammensetzung, zur Anwendung bei Kindern) (nicht erfindungsgemäß):

| **Inhaltsstoff** | **Menge/mg** | **Qualität** |
|---|---|---|
| Oxymetazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.407,00 | Ph. Eur. |

Rezeptur 5 (Angabe pro 10 g Zusammensetzung, zur Anwendung bei Erwachsenen):

| **Inhaltsstoff** | **Menge/mg** | | **Qualität** |
|---|---|---|---|
| Xylometazolinhydrochlorid | 10,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydrogenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Gereinigtes Wasser | 9.398,00 | | Ph. Eur. |

Rezeptur 6 (Angabe pro 10 g Zusammensetzung, zur Anwendung bei Erwachsenen) (nicht erfindungsgemäß):

| **Inhaltsstoff** | **Menge/mg** | | **Qualität** |
|---|---|---|---|
| Oxymetazolinhydrochlorid | 10,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydroaenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Gereinigtes Wasser | 9.398,00 | | Ph. Eur. |

Rezeptur 7 (Angabe pro 10 g Zusammensetzung, zur Anwendung bei Kindern):

| **Inhaltsstoff** | **Menge/mg** | | **Qualität** |
|---|---|---|---|
| Xylometazolinhydrochlorid | 5,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydrogenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Gereinigtes Wasser | 9.403,00 | | Ph. Eur. |

Rezeptur 8 (Angabe pro 10 g Zusammensetzung, zur Anwendung bei Kindern) (nicht erfindungsgemäß):

| **Inhaltsstoff** | **Menge/mg** | | **Qualität** |
|---|---|---|---|
| Oxymetazolinhydrochlorid | 5,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydrogenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Gereinigtes Wasser | 9.403,00 | | Ph. Eur. |

Rezeptur 9 (Angabe pro 10 g Zusammensetzung, zur Anwendung bei Erwachsenen):

| **Inhaltsstoff** | **Menge/mg** | | **Qualität** |
|---|---|---|---|
| Xylometazolinhydrochlorid | 10,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydroaenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Hyaluronsäure (Natriumsalz) | 50,00 | | |
| Natriumchlorid | 90,00 | | |
| Hydroxyectoin | 50,00 | | |
| Gereinigtes Wasser | 9.208,00 | | Ph. Eur. |

Rezeptur 10 (Angabe pro 10 g Zusammensetzung, zur Anwendung bei Kindern):

| **Inhaltsstoff** | **Menge/mg** | | **Qualität** |
|---|---|---|---|
| Xylometazolinhydrochlorid | 5,00 | | Ph. Eur. |
| Dexpanthenol | 500,00 | | Ph. Eur. |
| Puffersystem: | | | Ph. Eur. |
| Kaliumdihydrogenphosphat / | 85,30 | | |
| Natriummonohydroaenphosphat (Dodekahydrat) | 2,70 | | |
| Konservierungs-/Desinfektionsmittel: | 4,00 | | Ph. Eur. |
| Benzalkoniumchlorid (als 50 %-ige Lösung) | | | |
| Hyaluronsäure (Natriumsalz) | 50,00 | | |
| Natriumchlorid | 90,00 | | |
| Hydroxyectoin | 50,00 | | |
| Gereinigtes Wasser | 9.213,00 | | Ph. Eur. |

**2. Anwendungsbeobachtungen und klinische Untersuchungen:**
Je 20 Probanden eines aus 100 Patienten bestehenden Probandenkollektivs im Alter von 28 bis 79 Jahren (56 männlich, 44 weiblich), welche an akuten Rhinitiden litten, wurden mit den für die Erwachsenentherapie bestimmten Rezepturen 1 bzw. 2 bzw. 5 bzw. 6 bzw. 9 für die Dauer der Erkrankung (im Allgemeinen 3 bis 10 Tage) behandelt; die Rezepturen wurden mehrmals täglich als Spray topisch appliziert. Der vasokonstriktorische Effekt von Xylometazolinhydrochlorid bzw. Oxymetazolinhydrochlorid konnte, bezogen auf die Verbesserung der Symptomatik der Nasenschwellung und Behinderung der Nasenatmung, bei allen Patienten signifikant aufgezeigt werden. Aufgrund des Vorhandenseins der Dexpanthenolkomponente sowie gegebenenfalls weiterer Komponenten (z. B. Hyaluronsäure bzw. Hydroxyectoin bzw. Natriumchlorid) wurden keinerlei Reizwirkungen der Nasenschleimhäute infolge des Xylometazolinhydrochlorids bzw. Oxymetazolinhydrochlorids beobachtet. Insbesondere wurden auch keine Irritationen der Nasenschleimhäute infolge eines sogenannten "Rebound-Effektes", wie er bei Einsatz von Xylometazolinhydrochlorid bzw. Oxymetazolinhydrochlorid als alleinigem Wirksubstanz (d. h. ohne das Vorhandensein von Dexpanthenol) nach wiederholter Anwendung üblicherweise auftritt, beobachtet.
In analoger Weise und mit vergleichbarem Behandlungserfolg wurden je 5 Probanden eines aus 25 Patienten bestehenden Probandenkollektivs im Alter von 3 bis 12 Jahren (14 männlich, 11 weiblich), welche gleichermaßen an akuten Rhinitiden litten, mit den für die Kindertherapie bestimmten Rezepturen 3 bzw. 4 bzw. 7 bzw. 8 bzw. 10 für die Dauer der Erkrankung (im Allgemeinen ebenfalls 3 bis 10 Tage) behandelt.
Weitergehende klinischen Untersuchungen sowie weiterführende randomisierte placebokontrollierte Doppelblindstudien der Anmelderin bestätigen darüber hinaus einen Synergismus der Wirkungen der Wirkstoffe der erfindungsgemäßen Zusammensetzungen, was bei der Behandlung von Rhinitiden zu einer klinisch deutlicheren Besserung führt, und zwar über das Ausmaß der Einzelwirkstoffe deutlich hinausgehend.

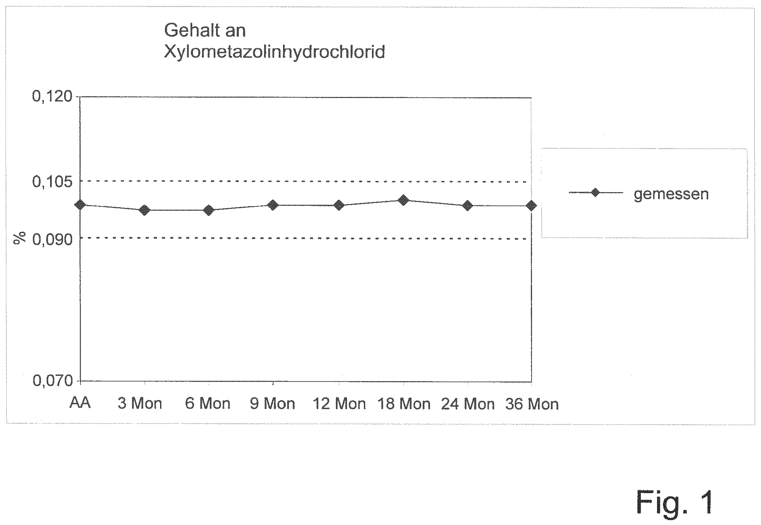

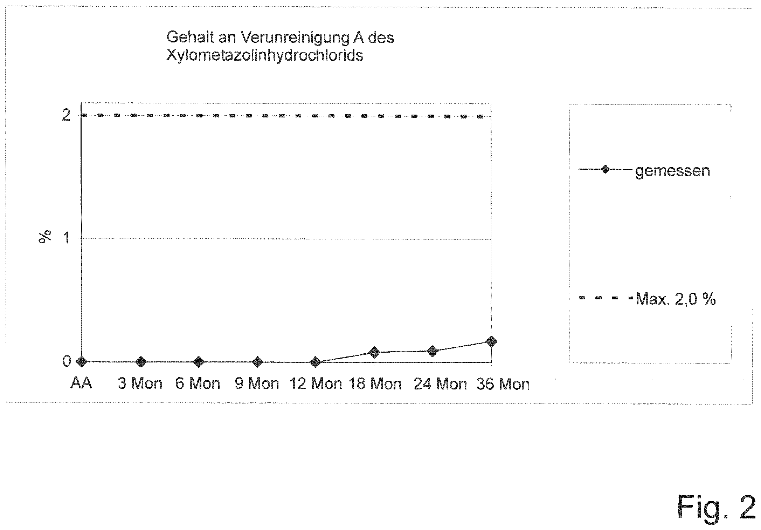

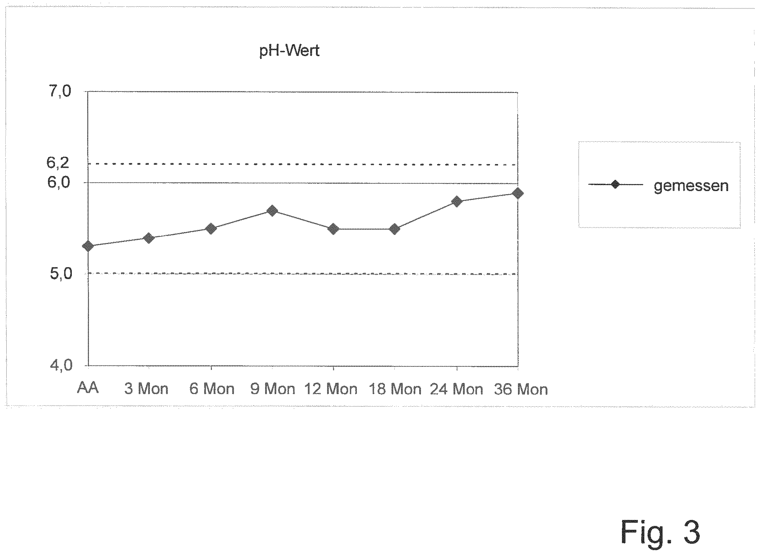

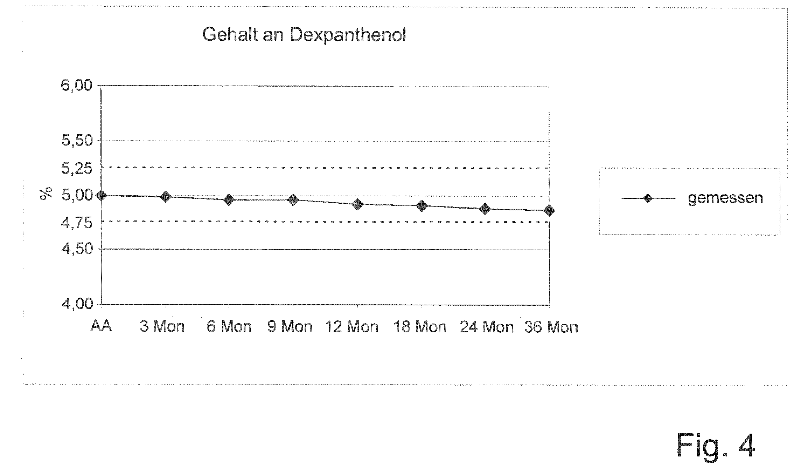

**3. Stabilitätsuntersuchungen:**
Nachfolgend sind die Ergebnisse einer Vielzahl von Stabilitätsuntersuchungen wiedergegeben.
Zu diesem Zweck wurden jeweils unter definierten Lagerbedingungen, wie sie nachstehend jeweils angegeben sind, Langzeitstabilitätsuntersuchungen an den betreffenden Zusammensetzungen durchgeführt, wobei nach definierten Zeiträumen spezielle Prüfparameter an den Zusammensetzungen gemessen und mit der betreffenden Spezifikation verglichen wurden (z. B. Aussehen der Zusammensetzung, pH-Wert, relative Dichte, Osmolalität sowie Gehalt an Wirkstoffen sowie an Verunreinigungen). Sofern nachstehend nicht ausdrücklich anders angegeben, erfolgte die Lagerung bei einer definierten Temperatur von 25 °C ± 2 °C und bei einer relativen Luftfeuchte der Umgebung von 60 % ± 5 %.
Ein charakteristisches Maß für die Stabilität einer Zusammensetzung ist zum einen die Konstanz des pH-Wertes sowie die Konstanz des Gehalts an Wirkstoffen (d. h. Xylometazolinhydrochlorid bzw. Oxymetazolinhydrochlorid sowie Dexpanthenol). Weiterhin kann der Gehalt an Abbauprodukten der Wirkstoffe bestimmt werden, um die Stabilität der Zusammensetzung zu bewerten.
Wie eingangs geschildert, ist die sogenannte Verunreinigung A ein typisches Abbauprodukt des Xylometazolinhydrochlorids, während als Abbauprodukte des Dexpanthenols Aminopropanol bzw. dessen Ammoniumsalz und D-Pantolacton charakteristisch sind. In der nachfolgenden Beschreibung der Ergebnisse der Stabilitätsuntersuchungen beziehen sich die Prozentangaben der Wirkstoffe Xylometazolinhydrochlorid bzw. Oxymetazolinhydrochlorid und Dexpanthenol auf Gewichtsprozentangaben mit Bezug auf die Zusammensetzung, während dagegen die prozentualen Gehaltsangaben aller Verunreinigungen jeweils als Gewichtsprozentangaben mit Bezug auf den ursprünglichen Wirkstoff bezogen sind, d. h. beispielsweise der Gehalt an Verunreinigung A als Gewichtsprozentangabe in Bezug auf Xylometazolinhydrochlorid und der Gehalt an Aminopropanol bzw. D-Pantolacton als Gewichtsprozentangabe in Bezug auf Dexpanthenol.
Mit anderen Worten beziehen sich bei den Verunreinigungen die Gewichtsprozentangaben gerade nicht auf die Zusammensetzung, sondern auf den Gehalt des betreffenden Wirkstoffs, aus dessen Abbau die betreffende Verunreinigung resultiert.
a) Stabilitätsuntersuchungen
An einer Charge einer erfindungsgemäßen Zusammensetzung gemäß der zuvor beschriebenen Rezeptur 1 wurden Stabilitätsuntersuchungen durchgeführt. Zu diesem Zweck wurde zum einen sowohl der Gehalt an Xylometazolinhydrochlorid (vgl. Fig. 1) als auch der Gehalt an dessen Abbauprodukt (Verunreinigung A) (vgl. Fig. 2) bestimmt, und zwar jeweils nach den in Fig. 1 und 2 angegebenen Lagerzeiten von 3 Monaten, 6 Monaten, 9 Monaten, 12 Monaten, 18 Monaten, 24 Monaten und 36 Monaten. Zum anderen wurde über denselben Lagerzeitraum für die vorgenannten Lagerzeiten auch der pH-Wert (vgl. Fig. 3) als auch der Gehalt an Dexpanthenol (vgl. Fig. 4) bestimmt. Die Lagerung erfolgte bei einer definierten Temperatur von 25 °C ± 2 °C und bei einer relativen Luftfeuchte der Umgebung von 60 % ± 5 %. Die erhaltenen Ergebnisse sind in den Figurendarstellungen gemäß Fig. 1 bis 4 dargestellt.
Fig. 1 zeigt den relativen Gehalt an Xylometazolin in Gewichtsprozent, bezogen auf die Zusammensetzung, über den Lagerzeitraum von 36 Monaten. Es wird eine ausgezeichnete Stabilität des Xylometazolinhydrochlorids beobachtet, d. h. es erfolgt im Wesentlichen kein Abbau.
Dies korreliert mit den Ergebnissen, wie sie in Fig. 2 dargestellt sind, wobei Fig. 2 den relativen Gehalt an Verunreinigungen A in Gewichtsprozent, bezogen auf das Xylometazolinhydrochlorid, über die Lagerzeit darstellt. Über die ersten 12 Monate Lagerzeit wird keinerlei Gehalt an Verunreinigung A festgestellt. Auch in den verbleibenden 24 Monaten steigt der Gehalt an Verunreinigung A nicht signifikant an und liegt weit unterhalb der tolerierbaren Spezifikationsgrenze von 2 Gew.-%, bezogen auf Xylometazolinhydrochlorid.
Wie Fig. 3 weiterhin zeigt, bleibt auch der pH-Wert der erfindungsgemäßen Zusammensetzung für den gesamten Lagerzeitraum von 36 Monaten deutlich innerhalb der Spezifikationsgrenzen zwischen 5,0 und 6,2.
Schließlich belegt auch Fig. 4 die Stabilität der erfindungsgemäßen Zusammensetzung, reflektiert am Gehalt an Dexpanthenol in der erfindungsgemäßen Zusammensetzung in Gewichtsprozent, bezogen auf die Zusammensetzung, wobei der betreffende Gehalt des Dexpanthenols geringfügig abnimmt über die gesamte Lagerzeit von 36 Monaten, aber immer noch deutlich innerhalb der Spezifikationsgrenzen zwischen 4,75 Gew.-% und 5,25 Gew.-%, bezogen auf die Zusammensetzung, bleibt.
Die Ergebnisse belegen die ausgezeichnete Langzeitstabilität der erfindungsgemäßen Zusammensetzung

b) Weitere Stabilitätsuntersuchungen
An einer weiteren Charge einer erfindungsgemäßen Zusammensetzung gemäß Rezeptur 1 wurde ebenfalls eine Langzeitstabilitätsuntersuchung durchgeführt. Die Ergebnisse sind in der Tabelle 1 wiedergegeben.
c) Einfluss verschiedener Parameter auf die Stabilität der Zusammensetzung
Einfluss des pH-Werts
Es wurden verschiedene Zusammensetzungen analog der Rezeptur 1 hergestellt, wobei jedoch in Abweichung von Rezeptur 1 der pH-Wert im Bereich von 4,5 bis 7,5 variiert wurde. Die Ergebnisse sind in Tabelle 2 zusammengefasst. Wie die Ergebnisse zeigen, tritt unterhalb eines pH-Werts von 5,0 und oberhalb eines pH-Werts von 6,2 eine rasche Zersetzung der erfindungsgemäßen Zusammensetzung ein. Im pH-Bereich zwischen 5,0 und 6,2 werden dagegen gute Ergebnisse erhalten. Besonders gute Ergebnisse lassen sich im pH-Bereich von 5,0 bis 6,0, insbesondere 5,1 bis 6,0, besonders 5,2 bis 5,9, erhalten.
Einfluss der Art des Puffersystems
Weiterhin wurden Untersuchungen mit unterschiedlichen Puffersystemen durchgeführt. Neben dem erfindungsgemäß eingesetzten Phosphatpuffersystem wurden auch Puffersysteme auf Basis von Kohlensäure/Bicarbonat, Essigsäure/Acetat, Kohlensäure/Silikat und Citronensäure/Citrat untersucht. Der Anfangs-pH-Wert aller Zusammensetzungen wurde auf ca. 5,5 eingestellt. Nur mit dem erfindungsgemäß verwendeten Phosphatpuffersystem lässt sich eine zuverlässige Langzeitstabilität erreichen. Die Ergebnisse sind in Tabelle 3 dargestellt.
Einfluss der Anwesenheit von Konservierungs- bzw. Desinfektionsmitteln
Weiterhin wurde der Einfluss von Konservierungs- bzw. Desinfektionsmitteln in Bezug auf die Stabilität der Zusammensetzung untersucht. Wie die Untersuchungen zeigen, wird durch die Anwesenheit eines Konservierungs- bzw. Desinfektionsmittels (Benzalkoniumchlorid) die Langzeitstabilität der Zusammensetzung noch weiterführend verbessert. Die betreffenden Ergebnisse sind in der Tabelle 4 wiedergegeben.
Einfluss des Dihydrogenphosphat/Monohydrogenposphat-Molverhältnisses
Weiterhin wurde der Einfluss des Dihydrogenphosphat/Monohydrogenposphat-Molverhältnisses untersucht. Nur mit dem erfindungsgemäß gewählten Molverhältnis lässt sich eine besonders effiziente Langzeitstabilisierung der Zusammensetzung erhalten.
Wie die vorstehend referierten Ergebnisse der Stabilitätsuntersuchungen der Anmelderin belegen, bedurfte es zur Auffindung einer stabilen Zusammensetzung für die Behandlung von Rhinitiden nach Art der vorliegenden Erfindung eines intensiven Forschungsaufwands seitens der Anmelderin.
Die vorstehend referierten Stabilitätsuntersuchungen zeigen die Komplexität des von der Anmelderin aufgefundenen Lösungsansatzes. Hierzu zählen die gemeinsame Anwesenheit der Wirkstoffe a) und b) in einem definierten Mengenverhältnis unter gleichzeitiger Einhaltung bzw. Konstanthaltung des pH-Werts der Zusammensetzung im zuvor definierten Bereich - ebenso wie weitere Cofaktoren, wie insbesondere die Auswahl des geeigneten Puffersystems sowie des geeigneten Molverhältnisses der Komponenten dieses Puffersystems. Eine zusätzliche Stabilitätsverbesserung lässt sich durch Konservierungs- und/oder Desinfektionsmittel, insbesondere auf Basis von Benzalkoniumchlorid, erreichen.
Der erfindungsgemäße Lösungsansatz kommt im Ergebnis in überraschender Weise mit nur wenigen zusätzlichen Inhaltsstoffen aus, welche aber die Wirkung der eigentlichen Wirkstoffe nicht beeinträchtigen; dies ist im Hinblick auf die beabsichtigte pharmazeutische Wirkung von entscheidender Bedeutung.

**Tabelle 1**

| **Prüfparameter** | **Spezifikation** | **Anfang** | **3 Monate** | **6 Monate** | **9 Monate** | **12 Monate** | **18 Monate** | **24 Monate** | **36 Monate** |
|---|---|---|---|---|---|---|---|---|---|
| **Aussehen** | farblose klare Flüssigkeit (Klarheit: < Ref.-Susp.; Färbung: < B9) | erfüllt | erfüllt | erfüllt | erfüllt | erfüllt | erfüllt | erfüllt | erfüllt |
| **Relat. Dichte (20 °C)** | 1,005-1,105 | 1,0153 | 1,0153 | 1,0153 | 1,0153 | 1,0154 | 1,0149 | 1,0155 | 1,0153 |
| **Osmolalität (mosm/kg)** | 350-495 | 413 | 420 | 414 | 417 | 425 | 429 | 427 | 430 |
| **Xylometazolinhydrochlorid (Gew.-%)** | 0,100 (0,095-0,105) | 0,1013 | 0,1036 | 0,1028 | 0,1020 | 0,1032 | 0,1017 | 0,1044 | 0,1015 |
| **Verunreinigung A (Gew.-%)** | max. 1,0 | n.b. | n.b. | 0,013 | 0,068 | 0,070 | 0,075 | 0,092 | 0,160 |
| **Dexpanthenol (Gew.-%)** | 5,00 (4,50-5,25) | 5,21 | 5,24 | 5,24 | 5,15 | 5,25 | 5,17 | 5,19 | 5,15 |
| **3-Aminopropanol (Gew.-%)** | max. 3,0 | 0,32 | 0,51 | 0,67 | 0,76 | 0,83 | 0,90 | 0,94 | 0,99 |
| **D-Pantolacton (Gew.-%)** | max. 3,0 | n.b. | n.b. | 0,0100 | 0,0186 | 0,0210 | 0,0330 | 0,0450 | 0,0600 |
| **Sterilität** | Ph.Eur. 5.1.4 2 Kat. 1 | erfüllt | erfüllt | erfüllt | erfüllt | erfüllt | erfüllt | erfüllt | erfüllt |

| | | | | | | | | | |
|---|---|---|---|---|---|---|---|---|---|
| n.b. = nicht bestimmbar (unterhalb der Analysegrenze) | | | | | | | | | |

**Tabelle 2**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **2A*** | **Xylometazolinhydrochlorid (Gew.-%)** | 0,100 (0,095-0,105) | 0,1011 | 0,1000 | 0,0998 | 0,0980 | 0,0945 | 0,0890 | 0,0802 |
| | **Verunreinig. A (Gew.-%)** | max. 1,0 | 0,071 | 0,441 | > 1 | > 1 | > 1 | > 1 | > 1 |
| | **Dexpanthenol (Gew.-%)** | 5,00(4,50-5,25) | 5,19 | 5,01 | 4,83 | 4,69 | < 4,50 | < 4,50 | < 4,50 |
| | **3-Aminopropanol(Gew.-%)** | max. 3,0 | 0,41 | 1,12 | > 3 | > 3 | > 3 | > 3 | > 3 |
| **2B*** | **Xylometazolinhydrochlorid (Gew.-%)** | 0,100 (0,095-0,105) | 0,1013 | 0,1029 | 0,1021 | 0,1022 | 0,1030 | 0,1019 | 0,1033 |
| | **Verunreinig. A (Gew.-%)** | max. 1,0 | n.b. | n.b. | 0,019 | 0,070 | 0,079 | 0,089 | 0,090 |
| | **Dexpanthenol (Gew.-%)** | 5,00(4,50-5,25) | 5,21 | 5,20 | 5,23 | 5,19 | 5,24 | 5,13 | 5,10 |
| | **3-Aminopropanol(Gew.-%)** | max. 3,0 | 0,35 | 0,45 | 0,67 | 0,81 | 0,93 | 1,02 | 1,12 |
| **2C** | **Xylometazolinhydrochlorid (Gew.-%)** | 0,100 (0,095-0,105) | 0,1013 | 0,1036 | 0,1028 | 0,1020 | 0,1032 | 0,1017 | 0,1044 |
| | **Verunreinig. A (Gew.-%)** | max. 1,0 | n.b. | n.b. | n.b. | 0,068 | 0,070 | 0,075 | 0,092 |
| | **Dexpanthenol (Gew.-%)** | 5,00(4,50-5,25) | 5,21 | 5,24 | 5,24 | 5,15 | 5,25 | 5,17 | 5,19 |
| | **3-Aminopropanol(Gew.-%)** | max. 3,0 | 0,29 | 0,37 | 0,52 | 0,65 | 0,75 | 0,82 | 0,99 |

| | | | | | | | | | |
|---|---|---|---|---|---|---|---|---|---|
| n.b. = nicht bestimmbar (unterhalb der Analysegrenze) * = nicht erfindungsgemäß | | | | | | | | | |

**Tabelle 2 (Fortsetzung)**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **2D** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.-%)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,31 | 0,43 | 0,51 | 0,63 | 0,72 | 0,88 | 0,98 |
| **2E*** | **Xylometazolinhydrochlorid (Gew.-%)** | 0,100 (0,095-0,105) | 0,1011 | 0,1000 | 0,1018 | 0,0999 | 0,0955 | 0,0901 | 0,0802 |
| | **Verunreinig. A (Gew.-%)** | max. 1,0 | 0,056 | 0,260 | 0,988 | > 1 | > 1 | > 1 | > 1 |
| | **Dexpanthenol (Gew.-%)** | 5,00(4,50-5,25) | 5,21 | 5,01 | 4,90 | 4,80 | 4,70 | < 4,50 | < 4,50 |
| | **3-Aminopropanol(Gew.-%)** | max. 3,0 | 0,15 | 0,99 | 1,90 | > 3 | > 3 | > 3 | > 3 |
| **2F*** | **Xylometazolinhydrochlorid (Gew.-%)** | 0,100 (0,095-0,105) | 0,1010 | 0,1000 | 0,1018 | 0,0999 | 0,0955 | 0,0901 | 0,0802 |
| | **Verunreinig. A (Gew.-%)** | max. 1,0 | 0,075 | 0,489 | > 1 | > 1 | > 1 | > 1 | > 1 |
| | **Dexpanthenol (Gew.-%)** | 5,00(4,50-5,25) | 5,20 | 4,99 | 4,80 | 4,60 | < 4,50 | < 4,50 | < 4,50 |
| | **3-Aminopropanol(Gew.-%)** | max. 3,0 | 0,22 | 1,20 | >3 | >3 | > 3 | > 3 | >3 |

| | | | | | | | | | |
|---|---|---|---|---|---|---|---|---|---|
| n.b. = nicht bestimmbar (unterhalb der Analysegrenze) * = nicht erfindungsgemäß Anfangs-pH-Wert Zusammens.2A: 4,0 / Anfangs-pH-Wert Zusammens.2B: 5,0 / Anfangs-pH-Wert Zusammens.2C: 5,2 Anfangs-pH-Wert Zusammens.2D: 5,5 / Anfangs-pH-Wert Zusammens.2E: 6,5 / Anfangs-pH-Wert Zusammens.2F: 7,5 / | | | | | | | | | |

**Tabelle 3**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **3A*** | **Xylometazolinhydrochlorid (Gew.-%)** | 0,100 (0,095-0,105) | 0,1013 | 0,1011 | 0,0997 | 0,0988 | 0,0942 | 0,0890 | 0,0800 |
| | **Verunreinig. A (Gew.-%)** | max. 1,0 | 0,039 | 0,342 | 0,663 | 0,897 | > 1 | > 1 | > 1 |
| | **Dexpanthenol (Gew.-%)** | 5,00(4,50-5,25) | 5,21 | 5,10 | 5,00 | 4,86 | 4,57 | < 4,50 | < 4,50 |
| | **3-Aminopropanol(Gew.-%)** | max. 3,0 | 0,19 | 1,29 | 2,30 | 2,99 | > 3 | > 3 | > 3 |
| **3B*** | **Xylometazolinhydrochlorid (Gew.-%)** | 0,100 (0,095-0,105) | 0,1013 | 0,1012 | 0,1010 | 0,1003 | 0,0995 | 0,0978 | 0,0922 |
| | **Verunreinig. A (Gew.-%)** | max. 1,0 | n.b. | 0,220 | 0,362 | 0,443 | 0,677 | > 1 | > 1 |
| | **Dexpanthenol (Gew.-%)** | 5,00(4,50-5,25) | 5,21 | 5,20 | 5,08 | 4,98 | 4,67 | < 4,50 | < 4,50 |
| | **3-Aminopropanol(Gew.-%)** | max. 3,0 | n.b. | 1,11 | 1,70 | 2,99 | > 3 | > 3 | > 3 |

| | | | | | | | | | |
|---|---|---|---|---|---|---|---|---|---|
| n.b. = nicht bestimmbar (unterhalb der Analysegrenze) * = nicht erfindungsgemäß | | | | | | | | | |

**Tabelle 3 (Fortsetzung)**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **3C** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.-%)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,33 | 0,41 | 0,52 | 0,63 | 0,77 | 0,87 | 0,95 |
| **3D*** | **Xylometazolinhydrochlorid (Gew.-%)** | 0,100 (0,095-0,105) | 0,1011 | 0,1009 | 0,1000 | 0,0999 | 0,0955 | 0,0940 | 0,0902 |
| | **Verunreinig. A (Gew.-%)** | max. 1,0 | 0,046 | 0,255 | 0,560 | 0,789 | 0,999 | > 1 | > 1 |
| | **Dexpanthenol (Gew.-%)** | 5,00(4,50-5,25) | 5,22 | 5,11 | 5,01 | 4,99 | 4,67 | < 4,50 | < 4,50 |
| | **3-Aminopropanol(Gew.-%)** | max. 3,0 | 0,15 | 0,99 | 1,90 | 2,90 | > 3 | > 3 | > 3 |
| **3E*** | **Xylometazolinhydrochlorid (Gew.-%)** | 0,100 (0,095-0,105) | 0,1012 | 0,1014 | 0,1011 | 0,1001 | 0,0990 | 0,0980 | 0,0902 |
| | **Verunreinig. A (Gew.-%)** | max. 1,0 | n.b. | 0,190 | 0,370 | 0,489 | 0,789 | 0,999 | > 1 |
| | **Dexpanthenol (Gew.-%)** | 5,00(4,50-5,25) | 5,21 | 5,19 | 5,11 | 5,00 | 4,78 | < 4,50 | < 4,50 |
| | **3-Aminopropanol(Gew.-%)** | max. 3,0 | n.b. | 0,99 | 1,90 | 2,90 | > 3 | > 3 | > 3 |

| | | | | | | | | | |
|---|---|---|---|---|---|---|---|---|---|
| n.b. = nicht bestimmbar (unterhalb der Analysegrenze) * = nicht erfindungsgemäß Puffer: 3A: Kohlensäure/Bicarbonat / 3B: Essigsäure/Acetat / 3C: Phosphatpuffer (Erfindung) 3D: Kohlensäure/Silikat / 3E:Citronensäure/Citrat | | | | | | | | | |

**Tabelle 4**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **4A** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.-%)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,33 | 0,43 | 0,52 | 0,66 | 0,73 | 0,87 | 0,98 |
| **4B** | **Xylometazolinhydrochlorid (Gew.-%)** | 0,100 (0,095-0,105) | 0,1013 | 0,1016 | 0,1016 | 0,1020 | 0,1029 | 0,1020 | 0,1015 |
| | **Verunreinig. A (Gew.-%)** | max. 1,0 | n.b. | n.b. | n.b. | n.b. | n.b. | 0,033 | 0,049 |
| | **Dexpanthenol (Gew.-%)** | 5,00(4,50-5,25) | 5,21 | 5,21 | 5,21 | 5,20 | 5,20 | 5,20 | 5,20 |
| | **3-Aminopropanol(Gew.-%)** | max. 3,0 | 0,32 | 0,42 | 0,51 | 0,64 | 0,73 | 0,86 | 0,97 |

| | | | | | | | | | |
|---|---|---|---|---|---|---|---|---|---|
| Zusammensetzung 4A: ohne Benzalkoniumchlorid Zusammensetzung 4B: wie Zusammensetzung 4A, jedoch mit Benzalkoniumchlorid (0,02 Gew.-%) | | | | | | | | | |

## Patentansprüche

1. Pharmazeutische Zusammensetzung mit verbesserter Stabilität für die topische intranasale Applikation für die Behandlung von Rhinitiden,
wobei die Zusammensetzung als wässrige Zusammensetzung vorliegt und
wobei die Zusammensetzung enthält:
a) mindestens ein imidazolinbasiertes alpha-Sympathomimetikum oder dessen physiologisch unbedenkliches Salz, wobei das imidazolinbasierte alpha-Sympathomimetikum ausgewählt ist aus Xylometazolin;
und
b) Pantothenol;
**dadurch gekennzeichnet,**
**dass** der pH-Wert der Zusammensetzung im Bereich von 5,2 bis 5,9 eingestellt und/oder konstantgehalten ist, wobei die Einstellung und/oder Konstanthaltung des pH-Werts der Zusammensetzung mittels mindestens eines chemischen Puffersystems erfolgt, wobei als chemisches Puffersystem ein Dihydrogenphosphat/Monohydrogenposphat-Puffersystem eingesetzt ist, wobei das Dihydrogenphosphat/Monohydrogenposphat-Puffersystem mit einem Dihydrogenphosphat/Monohydrogenposphat-Molverhältnis im Bereich von 6 : 1 bis 110 : 1 eingesetzt ist;
**dass** die Zusammensetzung einen Gehalt an Abbauprodukt(en) des Wirkstoffs a), insbesondere Verunreinigung A, von höchstens 5 Gew.-%, bezogen auf den Wirkstoff a), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten; und
**dass** die Zusammensetzung einen Gehalt an Abbauprodukt(en) des Wirkstoffs b), insbesondere Aminopropanol und/oder D-Pantolacton, von jeweils höchstens 5 Gew.-%, bezogen auf den Wirkstoff b), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten.

2. Zusammensetzung nach Anspruch 1, **dadurch gekennzeichnet,**
**dass** die Komponente a) ausgewählt ist aus Xylometazolin in Form von dessen physiologisch unbedenklichen Salzen, besonders bevorzugt in Form von dessen Hydrochloridsalz.

3. Zusammensetzung nach Anspruch 1 oder 2, **dadurch gekennzeichnet,**
**dass** die Zusammensetzung enthält:
a) Xylometazolin oder deren physiologisch unbedenkliche Salze, besonders bevorzugt in Form von dessen Hydrochloridsalz;
und
b) Pantothenol;
und/oder
**dass** die Zusammensetzung die Komponente a), bezogen auf die Zusammensetzung, in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält; und/oder
**dass** die Zusammensetzung die Komponente b), bezogen auf die Zusammensetzung, in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält; und/oder
**dass** die Zusammensetzung die Komponenten a) und b) in einem Mengenverhältnis von Komponente a) zu Komponente b) im Bereich von 1 : 10 bis 1 : 1.000, insbesondere 1 : 15 bis 1 : 500, vorzugsweise 1 : 20 bis 1 : 250, bevorzugt 1 :25 bis 1 :200, besonders bevorzugt 1 :30 bis 1 : 175, ganz besonders bevorzugt 1 : 40 bis 1 : 150, noch mehr bevorzugt 1 : 45 bis 1 : 125, enthält.

4. Zusammensetzung nach einem der vorangehenden Ansprüche, **dadurch gekennzeichnet,**
**dass** die Zusammensetzung das Dihydrogenphosphat/Monohydrogenposphat-Puffersystem mit einem Dihydrogenphosphat/Monohydrogenposphat-Molverhältnis im Bereich von 6 : 1 bis 105 : 1, ganz besonders bevorzugt im Bereich von 7 : 1 bis 100 : 1, enthält.

5. Zusammensetzung nach einem der vorangehenden Ansprüche, **dadurch gekennzeichnet, dass**
die Zusammensetzung c) mindestens ein Konservierungsmittel und/oder Desinfektionsmittel enthält, insbesondere wobei das Konservierungsmittel und/oder Desinfektionsmittel ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen und besonders bevorzugt Benzalkoniumchlorid ist und/oder insbesondere wobei die Zusammensetzung das Konservierungsmittel und/oder Desinfektionsmittel, bezogen auf die Zusammensetzung, in einer Menge von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, enthält; und/oder dass die Zusammensetzung d) mindestens ein vorzugsweise saures Glykosaminoglykan oder dessen physiologisch unbedenkliche Salze oder Derivate, insbesondere Hyaluronsäure oder deren physiologisch unbedenkliche Salze, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung; und/oder dass die Zusammensetzung e) Ectoin oder mindestens ein Ectoinderivat, insbesondere ein Hydroxyectoin, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung; und/oder
dass die Zusammensetzung f) Natriumchlorid enthält, insbesondere in einer Menge von 0,001 bis 5 Gew.-%, insbesondere 0,01 bis 2 Gew.-%, vorzugsweise 0,1 bis 1 Gew.-%, bezogen auf die Zusammensetzung; und/oder
dass die Zusammensetzung mindestens einen weiteren Inhaltsstoff aufweist, insbesondere ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

6. Zusammensetzung nach einem der vorangehenden Ansprüche, **dadurch gekennzeichnet,**
**dass** die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist; und/oder
**dass** die Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist; und/oder
**dass** die Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, lagerstabil ist.

7. Zusammensetzung nach einem der vorangehenden Ansprüche, **dadurch gekennzeichnet,**
**dass** die Zusammensetzung einen Gehalt an Abbauprodukt(en) des Wirkstoffs a), insbesondere Verunreinigung A, von höchstens 3 Gew.-%, vorzugsweise von höchstens 2 Gew.-%, besonders bevorzugt von höchstens 1 Gew.-%, bezogen auf den Wirkstoff a), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, und/oder
**dass** die Zusammensetzung einen Gehalt an Abbauprodukt(en) des Wirkstoffs b), insbesondere Aminopropanol und/oder D-Pantolacton, von jeweils höchstens 3 Gew.-%, vorzugsweise von jeweils höchstens 2 Gew.-%, besonders bevorzugt von jeweils höchstens 1 Gew.-%, bezogen auf den Wirkstoff b), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten.

8. Applikationsvorrichtung, insbesondere für die topische, insbesondere nasale, vorzugsweise intranasale Applikation, bevorzugt in Form eines Behältnisses mit Tropf- oder Sprüheinrichtung, enthaltend eine Zusammensetzung nach einem der vorangehenden Ansprüche.

9. Applikationsvorrichtung nach Anspruch 8, **dadurch gekennzeichnet,**
**dass** die Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl, vorzugsweise im Bereich von 75 µl bis 125 µl, aufweist; und/oder
**dass** die Applikationsvorrichtung ein Vorratsbehälter mit einem Volumen im Bereich von 5 ml bis 100 ml, insbesondere 10 ml bis 50 ml, aufweist.

10. Zusammensetzung nach einem der Ansprüche 1 bis 7 zur Verwendung bei der prophylaktischen und/oder kurativen topischen Behandlung von Rhinitiden, insbesondere *Rhinitis acuta.*

11. Zusammensetzung zur Verwendung nach Anspruch 10 zur Behandlung von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta.*

12. Zusammensetzung zur Verwendung nach Anspruch 10 oder 11, **dadurch gekennzeichnet, dass** die pharmazeutische Zusammensetzung nach einem der vorangehenden Ansprüche intranasal appliziert wird.

13. Verfahren zur Stabilisierung eines Wirkstoffes a) und eines Wirkstoffes b) in gemeinsamer wässriger Lösung einer pharmazeutischen Zusammensetzung für die topische intranasale Applikation für die Behandlung von Rhinitiden durch Einstellung und/oder Konstanthalten des pH-Wertes im Bereich von 5,2 bis 5,9,
wobei die Zusammensetzung enthält:
a) mindestens ein imidazolinbasiertes alpha-Sympathomimetikum oder dessen physiologisch unbedenkliches Salz, wobei das imidazolinbasierte alpha-Sympathomimetikum ausgewählt ist aus Xylometazolin;
und
b) Pantothenol;
wobei der pH-Wert der Zusammensetzung im Bereich von 5,2 bis 5,9 eingestellt und/oder konstantgehalten wird, wobei die Einstellung und/oder Konstanthaltung des pH-Werts der Zusammensetzung mittels mindestens eines chemischen Puffersystems erfolgt, wobei als chemisches Puffersystem ein Dihydrogenphosphat/Monohydrogenposphat-Puffersystem eingesetzt wird, wobei das Dihydrogenphosphat/Monohydrogenposphat-Puffersystem mit einem Dihydrogenphosphat/Monohydrogenposphat-Molverhältnis im Bereich von 6 : 1 bis 110 : 1 eingesetzt wird;
wobei die Zusammensetzung einen Gehalt an Abbauprodukt(en) des Wirkstoffs a), insbesondere Verunreinigung A, von höchstens 5 Gew.-%, bezogen auf den Wirkstoff a), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten; und
wobei die Zusammensetzung einen Gehalt an Abbauprodukt(en) des Wirkstoffs b), insbesondere Aminopropanol und/oder D-Pantolacton, von jeweils höchstens 5 Gew.-%, bezogen auf den Wirkstoff b), enthält, auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten.

## Claims

1. Pharmaceutical composition with improved stability for topical intranasal application for the treatment of rhinitis,
wherein the composition is present as an aqueous composition and
wherein the composition contains:
a) at least one imidazoline-based alpha-sympathomimetic or a physiologically acceptable salt thereof, wherein the imidazoline-based alpha-sympathomimetic is selected from xylometazoline;
and
b) pantothenol;
**characterized**
**in that** the pH of the composition is adjusted and/or kept constant in the range of from 5.2 to 5.9, the pH of the composition being adjusted and/or kept constant by means of at least one chemical buffer system, the chemical buffer system used being a dihydrogen phosphate/monohydrogen posphate buffer system, the dihydrogen phosphate/mono hydrogen posphate buffer system having a dihydrogen phosphate/mono hydrogen posphate molar ratio in the range of from 6 : 1 to 110 : 1 is used;
that the composition contains a content of degradation product(s) of the active ingredient a), in particular impurity A, of at most 5% by weight, based on the active ingredient a), even after storage of the composition at temperatures in the range of from 20 °C to 50 °C, at a pressure of 1,013.25 mbar and at a relative humidity in the range of from 50% to 90% for at least 6 months; and
that the composition contains a content of degradation product(s) of the active ingredient b), in particular aminopropanol and/or D-pantolactone, of at most 5% by weight in each case, based on the active ingredient b), even after storage of the composition at temperatures in the range of from 20 °C to 50 °C, at a pressure of 1,013.25 mbar and at a relative humidity in the range of from 50% to 90% for at least 6 months.

2. Composition according to claim 1, **characterized**
**in that** component a) is selected from xylometazoline in the form of its physiologically acceptable salts, particularly preferably in the form of its hydrochloride salt.

3. Composition according to claim 1 or 2, **characterized**
**in that** the composition contains:
a) xylometazoline or physiologically safe salts thereof, particularly preferably in the form of its hydrochloride salt;
and
b) pantothenol;
and/or
**in that** the composition contains component a), based on the composition, in an amount of 0.001 to 2% by weight, in particular 0.005 to 1.5% by weight, preferably 0.01 to 1.2% by weight, preferably 0.02 to 1.0% by weight, particularly preferably 0.03 to 0.5% by weight, very particularly preferably 0.04 to 0.2% by weight; and/or
**in that** the composition contains component b), based on the composition, in an amount of 0.01 to 10% by weight, in particular 0.1 to 9% by weight, preferably 0.5 to 8% by weight, preferably 1 to 7% by weight, particularly preferably 2 to 6% by weight, very particularly preferably 3 to 6% by weight; and/or
**in that** the composition contains components a) and b) in a quantitative ratio of component a) to component b) in the range of from 1 : 10 to 1 : 1,000, in particular 1:15 to 1 : 500, preferably 1 : 20 to 1 : 250, preferably 1 : 25 to 1 : 200, particularly preferably 1 : 30 to 1 : 175, very particularly preferably 1 : 40 to 1 : 150, even more preferably 1 : 45 to 1 : 125.

4. Composition according to one of the preceding claims, **characterized in that** the composition contains the dihydrogen phosphate/monohydrogen phosphate buffer system with a dihydrogen phosphate/monohydrogen phosphate molar ratio in the range of from 6 : 1 to 105 : 1, most preferably in the range of from 7 : 1 to 100 : 1.

5. Composition according to one of the preceding claims, **characterized**
**in that** the composition c) contains at least one preservative and/or disinfectant, in particular wherein the preservative and/or disinfectant is selected from the group of (i) alkylbenzyldimethylammonium chlorides, in particular C₈-C₁₈-alkylbenzyldimethyl ammonium chlorides, and mixtures of various alkylbenzyldimethylammonium chlorides, preferably benzalkonium chloride, (ii) para-hydroxybenzoic acid esters and mixtures of various para-hydroxybenzoic acid esters, preferably nipa-esters, (iii) chlorhexidine as well as combinations of the aforementioned compounds and particularly preferably benzalkonium chloride, and/or in particular wherein the composition comprises the preservative and/or disinfectant, based on the composition, in an amount of 0.001 to 10% by weight.-% by weight, in particular 0.005 to 5% by weight, preferably 0.01 to 2% by weight, preferably 0.01 to 1% by weight, particularly preferably 0.01 to 0.5% by weight; and/or
**in that** the composition d) contains at least one preferably acidic glycosaminoglycan or physiologically acceptable salts or derivatives thereof, in particular hyaluronic acid or physiologically acceptable salts thereof, in particular in an amount of from 0.0001 to 10% by weight, in particular from 0.001 to 5% by weight, preferably from 0.01 to 2% by weight, based on the composition; and/or
**in that** the composition e) contains ectoine or at least one ectoine derivative, in particular a hydroxyectoine, in particular in an amount of from 0.0001 to 10% by weight, in particular from 0.001 to 5% by weight, preferably from 0.01 to 2% by weight, based on the composition; and/or
**in that** the composition contains f) sodium chloride, in particular in an amount of 0.001 to 5% by weight, in particular 0.01 to 2% by weight, preferably 0.1 to 1% by weight, based on the composition; and/or
**in that** the composition comprises at least one further ingredient, in particular selected from the group of processing aids, stabilizers, emulsifiers, antioxidants, humectants, thickeners, antiseptics, colorants, flavorings, fragrances, extenders, binders, wetting agents, vitamins, trace elements, minerals, micronutrients and/or essential oils and combinations thereof.

6. Composition according to one of the preceding claims, **characterized**
**in that** the composition has an osmolality in the range of from 300 to 600 mosm/kg, in particular in the range of from 310 to 550 mosm/kg, preferably in the range of from 300 to 525 mosm/kg, preferably in the range of from 325 to 510 mosm/kg, particularly preferably in the range of from 350 to 500 mosm/kg; and/or
**in that**, at a temperature of 20 °C and at a pressure of 1,013.25 mbar, the composition has a relative density, based on pure water, in the range of from 1.001 to 1.2, in particular in the range of from 1.005 to 1.15, preferably in the range of from 1.005 to 1.105; and/or
**in that** the composition is storage-stable at temperatures in the range from 20 °C to 50 °C, at a pressure of 1,013.25 mbar and at a relative humidity in the range of from 50% to 90% for at least 6 months, in particular at least 12 months, preferably at least 24 months, preferably at least 36 months.

7. Composition according to one of the preceding claims, **characterized**
**in that** the composition contains a content of degradation product(s) of the active ingredient a), in particular impurity A, of at most 3% by weight, preferably of at most 2% by weight, particularly preferably of at most 1%, based on the active ingredient a), in particular also after storage of the composition at temperatures in the range of from 20 °C to 50 °C, at a pressure of 1,013.25 mbar and at a relative humidity in the range of from 50% to 90% for at least 6 months, in particular at least 12 months, preferably at least 24 months, preferably at least 36 months, and/or
**in that** the composition contains a content of degradation product(s) of the active ingredient b), in particular aminopropanol and/or D-pantolactone, of at most 3% by weight, preferably of at most 2% by weight, particularly preferably of at most 1%, based on the active ingredient b), in particular also after storage of the composition at temperatures in the range of from 20 °C to 50 °C, at a pressure of 1,013.25 mbar and at a relative humidity in the range of from 50% to 90% for at least 6 months, in particular at least 12 months, preferably at least 24 months, preferably at least 36 months.

8. Application device, in particular for topical, in particular nasal, preferably intranasal application, preferably in the form of a container with drip or spray device, containing a composition according to one of the preceding claims.

9. Application device according to claim 8, **characterized**
**in that** the application device has a spraying device for uniformly applying the composition in a quantity per spray in the range of from 25 µl to 300 µl, in particular in the range of from 50 µl to 200 µl, preferably in the range of from 75 µl to 125 µl; and/or
**in that** the application device has a storage container with a volume in the range of from 5 ml to 100 ml, in particular 10 ml to 50 ml.

10. Composition according to any one of claims 1 to 7 for use in the prophylactic and/or curative topical treatment of rhinitis, in particular *rhinitis acuta.*

11. Composition for use according to claim 10 for the treatment of *rhinitis acuta, rhinitis allergica, rhinitis atrophicans, rhinitis hyperplastica* or *hypertrophicans, rhinitis mutilans, rhinitis nervosa* or *vasomotorica* or *rhinitis pseudomembranacea,* preferably *rhinitis acuta.*

12. Composition for use according to claim 10 or 11, **characterized**
**in that** the pharmaceutical composition according to one of the preceding claims is applied intranasally.

13. Process for stabilizing an active ingredient a) and an active ingredient b) in a common aqueous solution of a pharmaceutical composition for topical intranasal application for the treatment of rhinitis by adjusting and/or maintaining the pH in the range of from 5.2 to 5.9,
wherein the composition contains:
a) at least one imidazoline-based alpha-sympathomimetic or a physiologically acceptable salt thereof, wherein the imidazoline-based alpha-sympathomimetic is selected from xylometazoline;
and
b) pantothenol;
the pH of the composition being adjusted and/or kept constant in the range of from 5.2 to 5.9, the pH of the composition being adjusted and/or kept constant by means of at least one chemical buffer system, a dihydrogen phosphate/monohydrogen posphate buffer system being used as the chemical buffer system, the dihydrogen phosphate/mono hydrogen posphate buffer system having a dihydrogen phosphate/mono hydrogen posphate molar ratio in the range of from 6 : 1 to 110 : 1 is used;
wherein the composition contains a content of degradation product(s) of the active ingredient a), in particular impurity A, of at most 5% by weight, based on the active ingredient a), even after storage of the composition at temperatures in the range of from 20 °C to 50 °C, at a pressure of 1,013.25 mbar and at a relative humidity in the range of from 50% to 90% for at least 6 months; and
wherein the composition contains a content of degradation product(s) of the active ingredient b), in particular aminopropanol and/or D-pantolactone, of at most 5% by weight in each case, based on the active ingredient b), even after storage of the composition at temperatures in the range of from 20 °C to 50 °C, at a pressure of 1,013.25 mbar and at a relative humidity in the range of from 50% to 90% for at least 6 months.

## Revendications

1. Composition pharmaceutique à stabilité améliorée pour l'application intranasale topique pour le traitement des rhinites,
où la composition se présente sous la forme d'une composition aqueuse et
où la composition contient
a) au moins un alpha-sympathomimétique à base d'imidazoline ou un sel physiologiquement acceptable de celui-ci, où l'alpha-sympathomimétique à base d'imidazoline est choisi parmi la xylométazoline;
et
b) le pantothénol;
**caractérisé**
**en ce que** la valeur du pH de la composition est ajustée et/ou maintenue constante dans la plage de 5,2 à 5,9, l'ajustement et/ou le maintien constant de la valeur du pH de la composition s'effectuant au moyen d'au moins un système tampon chimique, un système tampon dihydrogénophosphate/monohydrogénophosphate étant utilisé comme système tampon chimique, le système tampon dihydrogénophosphate/mono hydrogénophosphate ayant un rapport molaire dihydrogénophosphate/mono hydrogénophosphate dans la plage de 6 : 1 à 110 : 1 est utilisé;
**en ce que** la composition contient une teneur en produit(s) de dégradation de la substance active a), en particulier en impureté A, d'au maximum 5 % en poids, par rapport à la substance active a), même après stockage de la composition à des températures dans la plage de 20 °C à 50 °C, à une pression de 1013,25 mbar et à une humidité relative dans la plage de 50 % à 90 % pendant au moins 6 mois; et
**en ce que** la composition contient une teneur en produit(s) de dégradation de la substance active b), en particulier en aminopropanol et/ou en D-pantolactone, de respectivement 5 % en poids au maximum par rapport à la substance active b), même après stockage de la composition à des températures dans la plage de 20°C à 50°C, à une pression de 1013,25 mbar et à une humidité relative de l'air dans la plage de 50 % à 90 % pendant au moins 6 mois.

2. Composition selon la revendication 1, **caractérisée**
**en ce que** le composant a) est choisi parmi la xylométazoline sous forme de ses sels physiologiquement acceptables, de manière particulièrement préférée sous forme de son sel de hydrochlorure (sel de chlorhydrate).

3. Composition selon la revendication 1 ou 2, **caractérisée**
**en ce que** la composition contient
a) la xylométazoline ou ses sels physiologiquement acceptables, de préférence sous forme de son sel de hydrochlorure (sel de chlorhydrate);
et
b) le pantothénol;
et/ou
**en ce que** la composition contient le composant a), par rapport à la composition, en une quantité de 0,001 à 2 % en poids, en particulier de 0,005 à 1,5 % en poids, de préférence de 0,01 à 1,2 % en poids, de préférence de 0,02 à 1,0 % en poids, de manière particulièrement préférée de 0,03 à 0,5 % en poids, de manière tout particulièrement préférée de 0,04 à 0,2 % en poids; et/ou
**en ce que** la composition contient le composant b), par rapport à la composition, en une quantité de 0,01 à 10 % en poids, en particulier de 0,1 à 9 % en poids, de préférence de 0,5 à 8 % en poids, de préférence de 1 à 7 % en poids, de manière particulièrement préférée de 2 à 6 % en poids, de manière tout particulièrement préférée de 3 à 6 % en poids; et/ou
que la composition contient les composants a) et b) dans un rapport quantitatif du composant a) au composant b) dans la plage de 1 : 10 à 1 : 1000, en particulier de 1 : 15 à 1 : 500, de préférence de 1 : 20 à 1 : 250, de préférence de 1 : 25 à 1 : 200, de manière particulièrement préférée de 1 : 30 à 1 : 175, tout particulièrement de 1 : 40 à 1 : 150, de manière encore plus préférée de 1 : 45 à 1 : 125.

4. Composition selon l'une quelconque des revendications précédentes, **caractérisée en ce que** la composition contient le système tampon dihydrogénophosphate/mono-hydrogénophosphate avec un rapport molaire dihydrogénophosphate/mono-hydrogénophosphate dans la plage de 6 : 1 à 105 : 1, de manière tout particulièrement préférée dans la plage de 7 : 1 à 100 : 1.

5. Composition selon l'une quelconque des revendications précédentes, **caractérisée**
**en ce que** la composition c) contient au moins un conservateur et/ou un désinfectant, en particulier où le conservateur et/ou le désinfectant est choisi dans le groupe constitué par (i) des chlorures d'alkylbenzyldiméthylammonium, en particulier des chlorures d'alkylbenzyldiméthyl ammonium en C_{S}-C_{1S}, et des mélanges de différents chlorures d'alkylbenzyldiméthylammonium, de préférence le chlorure de benzalkonium, (ii) des esters d'acide para-hydroxybenzoïque et des mélanges de différents esters d'acide para-hydroxybenzoïque, de préférence des esters de nipa, (iii) de la chlorhexidine ainsi que des combinaisons des composés susmentionnés et, de manière particulièrement préférée, du chlorure de benzalkonium et/ou, en particulier où la composition contient l'agent de conservation et/ou l'agent désinfectant, par rapport à la composition, en une quantité de 0,001 à 10 % en poids.-%, en particulier de 0,005 à 5 % en poids, de préférence de 0,01 à 2 % en poids, de préférence de 0,01 à 1 % en poids, de manière particulièrement préférée de 0,01 à 0,5 % en poids; et/ou
**en ce que** la composition d) contient au moins un glycosaminoglycane de préférence acide ou ses sels ou dérivés physiologiquement acceptables, en particulier l'acide hyaluronique ou ses sels physiologiquement acceptables, en particulier en une quantité de 0,0001 à 10 % en poids, en particulier de 0,001 à 5 % en poids, de préférence de 0,01 à 2 % en poids, par rapport à la composition; et/ou
**en ce que** la composition e) contient de l'ectoïne ou au moins un dérivé d'ectoïne, en particulier une hydroxyectoïne, notamment en une quantité de 0,0001 à 10 % en poids, en particulier de 0,001 à 5 % en poids, de préférence de 0,01 à 2 % en poids, par rapport à la composition; et/ou
**en ce que** la composition f) contient du chlorure de sodium, en particulier en une quantité de 0,001 à 5 % en poids, en particulier de 0,01 à 2 % en poids, de préférence de 0,1 à 1 % en poids, par rapport à la composition; et/ou
**en ce que** la composition présente au moins un autre ingrédient, en particulier choisi dans le groupe des auxiliaires technologiques, des stabilisateurs, des émulsifiants, des antioxydants, des humectants, des épaississants, des antiseptiques, des colorants, des arômes, des parfums, des substances odorantes, des diluants, des liants, des agents mouillants, des vitamines, des oligo-éléments, des substances minérales, des micronutriments et/ou des huiles essentielles ainsi que leurs combinaisons.

6. Composition selon l'une quelconque des revendications précédentes, **caractérisée**
**en ce que** la composition présente une osmolalité dans la plage de 300 à 600 mosm/kg, en particulier dans la plage de 310 à 550 mosm/kg, de préférence dans la plage de 300 à 525 mosm/kg, de préférence dans la plage de 325 à 510 mosm/kg, de manière particulièrement préférée dans la plage de 350 à 500 mosm/kg; et/ou
**en ce que** la composition présente, à une température de 20°C et à une pression de 1.013,25 mbar, une densité relative, par rapport à l'eau pure, dans la plage de 1,001 à 1,2, en particulier dans la plage de 1,005 à 1,15, de préférence dans la plage de 1,005 à 1,105; et/ou
**en ce que** la composition est stable au stockage à des températures dans la plage de 20°C à 50°C, à une pression de 1 013,25 mbar et à une humidité relative dans la plage de 50 % à 90 % pendant au moins 6 mois, en particulier pendant au moins 12 mois, de préférence pendant au moins 24 mois, de préférence pendant au moins 36 mois.

7. Composition selon l'une quelconque des revendications précédentes, **caractérisée**
**en ce que** la composition présente une teneur en produit(s) de dégradation de la substance active a), en particulier en impureté A, d'au plus 3 % en poids, de préférence d'au plus 2 % en poids, de manière particulièrement préférée d'au plus 1 % en poids par rapport à la substance active a).%, par rapport à la substance active a), en particulier également après stockage de la composition à des températures dans la plage de 20 °C à 50 °C, à une pression de 1 013,25 mbar et à une humidité relative dans la plage de 50 % à 90 % pendant au moins 6 mois, en particulier au moins 12 mois, de préférence au moins 24 mois, de préférence au moins 36 mois, et/ou
**en ce que** la composition présente une teneur en produit(s) de dégradation de la substance active b), en particulier en aminopropanol et/ou en D-pantolactone, de 3 % en poids au maximum dans chaque cas, de préférence de 2 % en poids au maximum dans chaque cas, de manière particulièrement préférée de 1 % en poids au maximum dans chaque cas, par rapport à la substance active b).%, par rapport à la substance active b), en particulier également après la mise en place de la composition à des températures dans la plage de 20°C à 50°C, à une pression de 1 013,25 mbar et à une humidité relative dans la plage de 50 % à 90 % d'au moins 6 mois, en particulier d'au moins 12 mois, de préférence d'au moins 24 mois, de préférence d'au moins 36 mois.

8. Dispositif d'application, en particulier pour l'application topique, en particulier nasale, de préférence intranasale, de préférence sous la forme d'un récipient avec un dispositif de goutte à goutte ou de pulvérisation, contenant une composition selon l'une des revendications précédentes.

9. Dispositif d'application selon la revendication 8, **caractérisé**
**en ce que** le dispositif d'application présente un dispositif de pulvérisation pour l'application uniforme de la composition en une quantité par coup de pulvérisation dans la plage de 25µ I à 300µ I, en particulier dans la plage de 50 µl à 200 µl, de préférence dans la plage de 75 µl à 125 µl; et/ou
**en ce que** le dispositif d'application présente un réservoir d'un volume dans la plage de 5 ml à 100 ml, en particulier de 10 ml à 50 ml.

10. Composition selon l'une quelconque des revendications 1 à 7, destinée à être utilisée dans le traitement topique prophylactique et/ou curatif de la rhinite (*rhinitis*), en particulier *rhinitis acuta.*

11. Composition à utiliser selon la revendication 10 pour le traitement de *rhinitis acuta, rhinitis allergica, rhinitis atrophicans, rhinitis hyperplastica* ou *hypertrophicans, rhinitis mutilans, rhinitis nervosa* ou *vasomotorica* ou *rhinitis pseudomembranacea*, de préférence *rhinitis acuta.*

12. Composition à utiliser selon la revendication 10 ou 11, **caractérisée en ce que** la composition pharmaceutique selon l'une quelconque des revendications précédentes est appliquée par voie intranasale.

13. Procédé de stabilisation d'un principe actif a) et d'un principe actif b) dans une solution aqueuse commune d'une composition pharmaceutique pour l'application intranasale topique pour le traitement des rhinites par ajustement et/ou maintien du pH dans la plage de 5,2 à 5,9,
où la composition contient
a) au moins un alpha-sympathomimétique à base d'imidazoline ou un sel physiologiquement acceptable de celui-ci, où l'alpha-sympathomimétique à base d'imidazoline est choisi parmi la xylométazoline;
et
b) le pantothénol;
la valeur du pH de la composition étant ajustée et/ou maintenue constante dans la plage de 5,2 à 5,9, l'ajustement et/ou le maintien constant de la valeur du pH de la composition s'effectuant au moyen d'au moins un système tampon chimique, un système tampon dihydrogénophosphate/monohydrogénophosphate étant utilisé comme système tampon chimique, le système tampon dihydrogénophosphate/mono hydrogénophosphate ayant un rapport molaire dihydrogénophosphate/mono hydrogénophosphate dans la plage de 6 : 1 à 110 : 1 est utilisé;
la composition contenant une teneur en produit(s) de dégradation de la substance active a), en particulier en impureté A, d'au plus 5 % en poids par rapport à la substance active a), même après stockage de la composition à des températures dans la plage de 20°C à 50°C, à une pression de 1 013,25 mbar et à une humidité relative dans la plage de 50 % à 90 % pendant au moins 6 mois; et
la composition contenant une teneur en produit(s) de dégradation de la substance active b), en particulier en aminopropanol et/ou en D-pantolactone, de respectivement 5 % en poids au maximum, par rapport à la substance active b), même après stockage de la composition à des températures dans la plage de 20°C à 50°C, à une pression de 1 013,25 mbar et à une humidité relative dans la plage de 50 % à 90 %, d'au moins 6 mois.
